(19) Europäisches Patentamt
European Patent Office
Office européen des brevets

(11) EP 0 787 824 B1

(12) EUROPEAN PATENT SPECIFICATION

(45) Date of publication and mention of the grant of the patent:
**29.09.2004 Bulletin 2004/40**

(51) Int Cl.7: **C23C 16/40**, A61J 1/00, C08J 7/04, B65D 23/08, B05D 7/00

(21) Application number: **97101065.7**

(22) Date of filing: **24.01.1997**

# (54) Container with a non-ideal barrier coating sequence composition

Behälter mit einer Sperrbeschichtung einer nicht idealer Schichtzusammensetzung

Récipient avec une couche barrière ayant une combinaison de compositions non idéale

(84) Designated Contracting States:
**DE ES FR GB IT**

(30) Priority: **30.01.1996 US 593978**

(43) Date of publication of application:
**06.08.1997 Bulletin 1997/32**

(73) Proprietor: **Becton Dickinson and Company**
**Franklin Lakes, New Jersey 07417-1880 (US)**

(72) Inventors:
- **Harvey, Noel G.**
  **Efland NC (US)**
- **Tropsha, Yelena G.**
  **Chapel Hill, NC 27514 (US)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(56) References cited:
**EP-A- 0 607 573** **WO-A-95/10117**
**DE-A- 4 404 690**

- **PATENT ABSTRACTS OF JAPAN vol. 016, no. 103 (M-1221), 13 March 1992 (1992-03-13) & JP 03 278946 A (OIKE IND CO LTD), 10 December 1991 (1991-12-10)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 787 824 B1

Printed by Jouve, 75001 PARIS (FR)

**Description**

## BACKGROUND OF THE INVENTION

### 1. Field of the Invention

This invention relates to a non-ideal barrier coating sequence composition for providing an effective barrier against gas and water permeability for containers, especially plastic blood collection tubes. In particular, the barrier coating composition of the present invention comprises the combination of thin films of inorganic oxides and organic polymers, such as highly crosslinked organic thermoset plastics. The composition of the present invention has improved barrier to oxygen and water vapor transport than would have been predicted from the barrier characteristics of either the individual oxide and organic polymer films.

### 2. Description of the Related Art

With the increased emphasis on the use of plastic medical products, a special need exists for improving the barrier properties of articles made of polymers.

Such medical products that would derive a considerable benefit from improving their barrier properties include, but are not limited to, collection tubes and particularly those used for blood collection.

Blood collection tubes require certain performance standards to be acceptable for use in medical applications. Such performance standards include the ability to maintain greater than about 90% original draw volume over a one year period, to be radiation sterilizable and to be non-interfering in tests and analysis.

Therefore, a need exists to improve the barrier properties of articles made of polymers and in particular plastic evacuated blood collection tubes wherein certain performance standards would be met and the article would be effective and usable in medical applications.

Glass-like or metal oxide films synthesized from chemical vapor deposition techniques have been used as thin barrier coatings on polypropylene films. However, glass-like thin films that are synthesized are substantially granular in morphology instead of substantially continuously glass-like and therefore do not have the oxygen and water vapor barrier characteristics of a truly continuous glass-material.

It has been shown that to overcome the drawbacks of the morphology of glass-like thin films is to "stack" layers of glass-like films with a continuous organic polymer film interposed between each layer. Such laminar multilayer coatings improve the oxygen barrier performance of polypropylene films, however such layering does not produce a glass-like barrier and the layering merely performs as a laminate of metal oxides and acrylate polymer coatings.

EP-A-0607573 is directed to a plastic container which finds utility in the medical field and which is coated with a barrier coating. The barrier coating comprises at least two layers of two distinct barrier materials, the first layer of which is an inorganic material and the second layer of which is a polymeric (=organic) material. The inorganic layer may preferably be aluminum oxide based. The second layer preferably is vinylidene chloride-acrylonitrile-methyl methacrylate-methyl acrylate-acrylic acid copolymers, thermosetting epoxy coatings, porylene polymers or polyesters.

It is therefore desirable to produce a composite that may be used to achieve gas and water barrier performance similar to glass.

## SUMMARY OF THE INVENTION

One aspect of the present invention is a plastic container having an open end, a closed end, an inner surface and an outer surface; comprising a non-ideal barrier coating sequence associated over the outer surface of

$$\sum_{n} (\text{organic material} + \text{inorganic material})$$

said container composition comprising: where n = 1-20,

wherein said inorganic material is deposited onto said organic material;

and wherein an organic material selected from poly(vinylidenechloride), vinylidene chloride-methyl methacrylate-methacrylate acrylic acid polymer (PVDC), vinylidene chloride-acrylonitrile-methyl methacrylate-methylacrylate-acrylic acid copolymers, thermosetting epoxy materials, parylene polymers or polyesters is disposed over the non-ideal barrier coating sequence.

Another aspect of the present invention is a method of depositing a non-ideal barrier coating sequence composition on the outer surface of a plastic container comprising:

(a) selecting a curable component comprising: i) polyfunctional acrylates, or ii) mixtures of monoacrylates and polyfunctional acrylates;
(b) flash vaporizing said component into said chamber;
(c) condensing an acrylate material of said vaporized component onto the outer surface of said container;
(d) curing said acrylate material;
(e) vaporizing an organosilicon component and admixing a volatilized organosilicon component with an oxidizer component and optionally an inert gas component to form a gas steam exterior to the chamber;
(f) establishing a glow discharge plasma in the chamber from one or more of the gas stream components;
(g) controllably flowing the gas stream into the plasma while confining at least a portion of the plasma therein;
(h) depositing a material of silicon oxide adjacent said acrylate material;
(i) repeating steps (a) through (d) above, thereby depositing an acrylate material on said silicon oxide material; and
(j) repeating steps (e) through (h) above, thereby depositing a silicon oxide material on said acrylate material.
(k) removing the container from the chamber and disposing an organic material selected from poly(vinylidenechloride), vinylidene chloride-methyl methacrylate-methacrylate acrylic acid polymer (PVDC), vinylidene chloride-acrylonitrile-methyl methacrylate-methylacrylate-acrylic acid copolymers, thermosetting epoxy materials, parylene polymers or polyesters over the non-ideal barrier coating sequence.

Preferred embodiments will become evident from the dependent claims.

Desirably, an organic material is preferably a highly cross linked acrylate or acrylic polymer.

Preferably, the organic material is a blend of monoacrylate (i.e., isobornyl acrylate) and diacrylate monomers (i.e., an epoxy diacrylate or a urethane diacrylate) as described in U.S. -A- 4,490,774, 4,696,719, 4,647,818, 4,842,893, 4,954,371 and 5,032,461, The organic material is cured by an electron beam or by a source of ultraviolet radiation.

Most preferably, the organic material is formed of a substantially cross linked component selected from the group consisting of polyacrylates and mixtures of polyacrylates and monacrylates having an average molecular weight of between 150 and 1,000 and a vapor pressure in the range of 0.133 to 13332 mPa ($1x10^{-6}$ to $1x10^{-1}$ Torr) at standard temperature and pressure. Most preferably, the material is a diacrylate.

The organic material provides a platform for deposition of the inorganic material. Preferably, the thickness of the acrylate material is about 0.1 μm to 10 μm and most preferably from 0.5 μm to 3 μm.

Desirably, the inorganic material is a metal oxide, such as a silicon oxide based composition, such as $SiO_x$ wherein x is from 1.0 to 2.5; or an aluminium oxide based composition. Most preferably, the organic material is a highly cross linked acrylate polymer.

The silicon oxide based composition is substantially dense and vapor-impervious and is desirably derived from volatile organosilicon compounds and acrylate. Preferably, the thickness of the silicon oxide based material is 10 to 200 nm (100 to 2,000 Angstroms (Å)) and most preferably from 50 to 100nm (500 to 1,000 Å). A material above 500nm 5,000 Å) may crack and therefore be ineffective as a barrier.

An optional organic material may be disposed over the non-ideal barrier coating sequence composition and preferably comprises vinylidene chloride - methyl methacrylate - methacrylate acrylic acid polymer (PVDC), thermosetting epoxy materials, parylene polymers or polyester.

Preferably, the thickness of the PVDC material is 2 to 15 μm and most preferably from 3 to 5 μm.

The process for applying the organic material of the sequence is preferably carried out in a vacuum chamber wherein a curable monomer component is metered to a heated vaporizer system where the material is atomized, vaporized and condensed on the surface of the container. Following deposit of the monomer onto the surface of the container, it is cured by suitable means such as electron beam curing. The deposition and curing steps may be repeated until the desired thickness of materials has been achieved.

A method for depositing a silicon oxide based material is as follows: (a) pretreating the organic material on the container with a first plasma material of oxygen; (b) controllably flowing a gas stream including an organosilicon compound into a plasma; and (c) depositing a silicon oxide onto the organic material while maintaining a pressure of less than 66.7 Pa (500 mTorr) during the depositing.

The organosilicon compound is preferably combined with oxygen and optionally helium or another inert gas such as argon or nitrogen and at least a portion of the plasma is preferably magnetically confined adjacent to the surface of the organic material during the depositing, most preferably by an unbalanced magnetron.

Although the pretreatment step is optional, it is believed that the oxygen plasma pretreatment step provides for improved adherence qualities between the organic material and the organic material.

The PVDC material is optionally applied over the non-ideal barrier coating sequence composition by dipping or spraying and then followed by air drying at 50° C.

Most preferably, the method for depositing non-ideal a barrier coating sequence composition on a substrate, such as a plastic collection tube comprises the following steps:

(a) selecting a curable component comprising: i) polyfunctional acrylates, or ii) mixtures of monoacrylates and polyfunctional acrylates;
(b) flash vaporizing the component into the chamber;
(c) condensing an organic material of a vaporized component onto the outer surface of the container;
(d)curing the organic material;
(e) vaporizing an organosilicon component and admixing the volatilized organosilicon component with an oxidizer component and optionally an inert gas component to form a gas steam exterior to the chamber;
(f) controllably flowing the gas stream into the chamber;
(g) establishing a glow discharge plasma in the chamber from the gas stream;
(h) depositing a silicon oxide material adjacent the organic material;
(i) repeating steps (a) through (d) above, thereby depositing an acrylate material adjacent the silicon oxide material;
(j) repeating steps (e) through (h) above; thereby depositing a silicon oxide material on said acrylate material; and
(k) dip coating PVDC on the non-ideal barrier coating composition sequence.

Optionally, steps (i) through (j) may be repeated from about 1 to about 20 times before dip coating PVDC on the silicon oxide material.
Optionally, the container and/or the organic material may be flame-treated or plasma oxygen treated or corona discharge treated prior to applying the inorganic material.
Plastic tubes coated with the non-ideal barrier coating sequence composition are able to maintain substantially far better vacuum retention, draw volume and mechanical integrity retention than previous tubes comprised of polymer compositions and blends thereof without an ideal barrier coating or of tubes comprising only an oxide material. In addition, the tube's resistance to impact is much better than that of glass. Most notably is the clarity of the non-ideal barrier coating sequence composition of the present invention and its durability to substantially withstand resistance to impact and abrasion.
Most preferably, the container of the present invention is a blood collection device. The blood collection device can be either an evacuated blood collection tube or a non-evacuated blood collection tube. The blood collection tube is desirably made of polyethylene terephthalate (PET), polypropylene (PP), polyethylene napthalate (PEN), polycarbonate (PC) or copolymers thereof.
In addition to blood collection devices, the non-ideal barrier coating sequence composition of the present invention may be used with polymer films wherein both sides of the film include the non-ideal composition of the present invention. Such films may be about 51µm (0.002") or less in thickness.
Printing may be placed on the non-ideal barrier coating sequence composition of the present invention. For example, a product identification, bar code, brand name, company logo, lot number, expiration date and other data and information may all be included on the non-ideal composition. Moreover, a matte finish or a corona discharged surface may be developed on the non-ideal composition so as to make the surface appropriate for writing additional information on the label. Furthermore, a pressure sensitive adhesive label may be placed over the non-ideal composition so as to accommodate various hospital over-labels, for example.
Preferably, the non-ideal composition of the present invention provides a transparent or colorless appearance and may have printed matter applied thereon.
An advantage is that the non-ideal composition of the present invention provides a reduction in the gas permeability of three-dimensional objects that has not been achieved with conventional or ideal compositions typically used with thin films.
The non-ideal composition of the present invention provides a reduction in permeation greater than is expected by standard permeation theory. Permeation thermodynamics demonstrates that the non-ideal composition of the present invention exhibits a more nearly "glass-like" property than a single-layer ideal $SiO_x$ composition Therefore, the non-ideal composition of the present invention provides an unpredicted barrier system.
It has been found that a highly crosslinked layer of acrylate improves the adhesion between a plastic surface and $SiO_x$ and overall improves the thermomechanical stability of the non-ideal barrier coating sequence composition. In addition, the acrylate material covers the particles and imperfections on the surface of a polymer and reduces the defect density in the non-ideal barrier coating sequence composition. The good bonding properties of the acrylate are also due to the fact that acrylate is polar and the polarity provides means for good bond formation between the $SiO_x$ and the acrylate. In addition, it enhances the bond formation between plastic tubes made of polypropylene and $SiO_x$. Thus, the present invention provides the means of substantially improving the barrier properties of polypropylene tubes.
A plastic blood collection tube coated with the non-ideal barrier coating sequence composition of the present invention will not interfere with testing and analysis that is typically performed on blood in a tube. Such tests include

but are not limited to, routine chemical analysis, biological inertness, hematology, blood chemistry, blood typing, toxicology analysis or therapeutic drug monitoring and other clinical tests involving body fluids. Furthermore, a plastic blood collection tube coated with the non-ideal barrier coating sequence composition is capable of being subjected to automated machinery such as centrifuges and may be exposed to certain levels of radiation in the sterilization process with substantially no change in optical or mechanical and functional properties.

It has also been found that the non-ideal barrier coating sequence composition of the present invention does not exhibit true laminate properties in accordance with the laminate equation.

As illustrated in FIG. 1. when two or more different barrier films are stacked, the permeation of small molecules through the multilayer laminate is generally described by the laminate equation:

$$(\Pi_{12}) = (\Pi_1^{-1} + \Pi_2^{-1})^{-1}$$

where $\Pi_1$, is the permeation rate through component layer 1, $\Pi_2$, is the permeation rate through component layer 2 and $\Pi_{12}$, is the permeation rate through the laminate of components 1 and 2. When the permeance of the individual components is known, the permeance of the total layer laminate of those components can be calculated and predicted.

However, the transport rate of permeants through the non-ideal barrier coating sequence composition of the present invention is lower than the permeation rate predicted by the laminate equation. Therefore, the non-ideal barrier coating sequence composition of the present invention is a non-ideal composite with unpredicted transport rate of permeants.

The performance of the non-ideal barrier coating sequence composition of the present invention is different from predicted laminate because permeation of gases through the non-ideal barrier coating sequence composition of the present invention requires the expenditure of more thermal energy than would be predicted from the laminate equation.

The laminate equation is therefore modified for the non-ideal barrier coating sequence composition of the present invention as follows:

$$\Pi_{oi} < (\Pi_o^{-1} + \Pi_i^{-1})^{-1}$$

where $\Pi_o$ is the permeation rate through the organic material of the sequence, $\Pi_i$ is the permeation rate through the inorganic material of the sequence and $\Pi_{oi}$ is the permeation rate through the laminate of the organic and inorganic materials. The transport rate of permeants is therefore less than expected from ideal additivity.

It can therefore be concluded that the transport rate of permeants of the non-ideal barrier: coating sequence composition or non-ideal composite composition of the present invention is not an additive effect. Therefore, non-ideal composite composites must be discovered for maximum permeance efficiency or performance and not predicted. Furthermore, the permeance properties of a non-ideal composite composition are not inherent.

When the transmission rate of a permeant, such as oxygen or water, through a barrier structure is obtained at several different temperatures, the thermodynamic energy necessary to transport the permeant completely through the barrier structure is obtained by the Arrhenius equation:

$$\ln Q = \ln Q_o - \Delta G/RT$$

where $\Delta G$ is the energy necessary to move one mole of permeant molecules through the barrier structure in cal/mole, R is the gas constant in cal/mole - degree, T is temperature in degrees Kelvin, Q is the permeant transmission rate and $Q_O$ is a constant unique to the structure. In practice, the transmission rate Q for oxygen transport through the barrier structure is obtained at several temperatures. Then the natural log of the transmission rate obtained at each temperature versus the reciprocal of each temperature is plotted. The slope of the resultant linear plot is the quantity $-\Delta G/R$, from which $\Delta G$ is obtained.

It has also been found that the non-ideal barrier coating sequence composition of the present invention results in the expenditure of more thermal energy ($\Delta G$) than that of any of the components of the composition or $\Delta G_T > \Delta G_A$, $\Delta G_B$, where T is the non-ideal barrier coating sequence composition and A and B are the organic and inorganic components of the non-ideal barrier coasting sequence composition. In contrast, a laminate or ideal composite composition will have a $\Delta G_T = \Delta G_A$ or $\Delta G_B$, whichever component (A or B) has the lowest permeance.

## DESCRIPTION OF THE DRAWING

FIG. 1 is a perspective view of a typical blood collection tube with a stopper.

FIG. 2 is a longitudinal sectional view of the tube of FIG. 1 taken along line 2-2.

FIG. 3 is a longitudinal sectional view of a tube-shaped container similar to the tube of FIG. 1 without a stopper, comprising the barrier coating composition.

FIG. 4 is a longitudinal sectional view of a tube-shaped container, similar to the tube of FIG. 1 with a stopper, comprising the barrier coating composition.

FIG. 5 is a longitudinal sectional view of a further embodiment of the invention illustrating the tube with a stopper similar to FIG. 1 and with the barrier coating composition encompassing both the tube and stopper thereof.

FIG. 6 illustrates an enlarged partially sectioned, diagram of a flash evaporator apparatus.

FIG. 7 illustrates a plasma deposition system.

FIG. 8 is the plot of the natural log of the transmission rates versus the reciprocal of the temperature for the measurements of Examples 1, 3 and 4 and Table 1.

FIG. 9 illustrates the draw volume loss for PET/$[Ac/Sio_x]_n$ Tubes at 40°C, 1 atm in accordance with the data reported in Table 4.

## DETAILED DESCRIPTION

The present invention may be embodied in other specific forms and is not limited to any specific embodiments described in detail which is merely exemplary. Various other modifications will be apparent to and readily made by those skilled in the art without departing from the scope and spirit of the invention. The scope of the invention will be measured by the appended claims and their equivalents.

Referring to the drawings in which like reference characters refer to like parts throughout the several views thereof, FIGS. 1 and 2 show a typical blood collection tube **10,** having a sidewall **11** extending from an open end **16** to a closed end **18** and a stopper **14** which includes a lower annular portion or skirt **15** which extends into and presses against the inner surface **12** of the sidewall for maintaining stopper **14** in place.

FIG. 2 schematically illustrates that there are three mechanisms for a change in vacuum in a blood collection tube: (A) gas permeation through the stopper material, (B) gas permeation through the tube and (C) leak at the stopper tube interface. Therefore, when there is substantially no gas permeation and no leak, there is good vacuum retention and good draw volume retention.

FIG. 3 shows the preferred embodiment of the invention, a plastic tube coated with a non-ideal barrier coating sequence composition. The preferred embodiment includes many components which are substantially identical to the components of FIGS. 1 and 2. Accordingly, similar components performing similar functions will be numbered identically to those components of FIGS. 1 and 2, except that a suffix "a" will be used to identify those components in FIG. 3.

Referring now to FIG. 3, the preferred embodiment of the invention, collection tube assembly **20** comprises a plastic tube **10a**, having a sidewall **11a** extending from an opened end **16a** to a closed end **18a**. A non-ideal barrier coating composition **25** extends over a substantival portion of the outer surface of the tube with the exception of open end **16a**. Non-ideal barrier coating sequence composition **25** comprises a sequence of organic and inorganic materials and PVDC.

The sequence composition preferably comprises multiple materials expressed as follows:

$$\text{Sequence} = \sum_{n} (\text{organic material} + \text{inorganic material})$$

where n=1-20.

FIG. 4 illustrates an alternate embodiment of the invention, wherein collection tube assembly **40** comprises stopper **48** in place for closing open end **41** of tube **42**. As can be seen, sidewall **43** extends from open end **41** to closed end **44** and stopper **48** includes an annular upper portion **50** which extends over the top edge of tube **42**. Stopper **48** includes a lower annular portion or skirt **49** which extends into and presses against the inside inner surface **46** of sidewall **43** for maintaining stopper **48** in place. Also, the stopper has a septum portion **52** for receiving a cannula therethrough.

Thus, the user, once receiving a container such as that shown in FIG. 4 with a sample contained therein, may insert a cannula through septum **52** for receiving part or all of the contents in tube **42** to perform various tests on a sample. Covering a substantial portion of the length of the tube is the non-ideal barrier coating sequence composition **25**. Non-ideal barrier coating sequence composition **25** covers substantially most of the tube with the exception of open end **41** thereof. FIG. 4 differs from the embodiment in FIG. 3 in that the tube may be evacuated with the simultaneous placement of stopper **48** therein after the application of non-ideal barrier coating sequence composition **25** over the tube. Alternatively, non-ideal barrier coating sequence composition **25** may be applied to the tube after it has been evacuated

FIG. 5 shows an additional embodiment of the non-ideal barrier coating sequence composition and a tube. The alternate embodiment functions in a similar manner to the embodiment illustrated in FIG. 4. Accordingly, similar components performing similar functions will be numbered identically to those components in the embodiment of FIG. 4, except that a suffix "a" will be used to identify those components in FIG. 5.

Referring now to FIG. 5, a further embodiment **60** of the invention wherein the non-ideal barrier coating sequence composition **25a** incorporates both upper portion **50a** of stopper **48a**, as well as the entire outer surface of tube **42a**. Non-ideal barrier coating sequence composition **25a** includes serrations **62** at the tube, stopper interface. The serrations are registered so that it can be determined if the sealed container has been tampered with. Such an embodiment may be utilized, for example, for sealing the container with the stopper in place. Once a sample has been placed in the tube, the sample cannot be tampered with by removal of the stopper. Additionally, the serrations may be registered so that it can be determined if the sealed container has been tampered with. Such an arrangement may be appropriate, for example, in drug abuse testing, specimen identification and quality control.

It will be understood by practitioners-in-the-art, that such tubes may contain reagents in the form of additives or coatings on the inner wall of the tube.

The non-ideal barrier coating sequence composition forms a substantially clear or translucent material. Therefore, the contents of a plastic tube with the non-ideal barrier coating sequence composition is substantially visible to the observer at the same time identifying information may be displayed over the non-ideal barrier coating sequence composition after it is applied to the plastic tube.

The organic material is an acrylate material and may be formed by dip-coating, roll-coating or spraying acrylate monomer or the blend of monomers, followed by UV curing process.

The acrylate material may also be applied by an evaporation and curing process carried out as described in U.S.-A-5,032,461.

The acrylate evaporation and curing process involves first atomizing the acrylate monomer into about 50 μm droplets and then flashing them off of a heated surface. This produces an acrylate molecular vapor which has the same chemistry as the starting monomer.

Acrylates are available with almost any chemistry desired. They usually have either one, two or three acrylate groups per molecule. Various mixtures of mono, di and tri acrylates are useful in the present invention. Most preferable are monoacrylates and diacrylates.

Acrylates form one of the most reactive classes of chemicals. They cure rapidly when exposed to UV or electron beam radiation to form a cross-linked structure. Therefore acrylates impart high temperature, stability and abrasion resistant properties to compositions.

The monomer materials utilized are relatively low in molecular weight, between 150 and 1,000 and preferably in the range of 200 to 300 and have vapor pressures between 0.133mPa ($1x10^{-6}$Torr) and 1.332 mPa ($1x10^{-1}$ Torr) at standard temperature and pressure (i.e., relatively low boiling materials). A vapor pressure of 1333 mPa ($1x10^{-2}$. Torr) is preferred. Polyfunctional acrylates are especially preferred. The monomers employed have at least two double bonds (i.e., a plurality of olefinic groups). The high-vapor-pressure monomers used in the present invention can be vaporized at low temperatures and thus are not degraded (cracked) by the heating process. The absence of unreactive degradation products means that films formed from these low molecular weight, high-vapor-pressure monomers have reduced volatile levels of components. As a result, substantially all of the deposited monomer is reactive and will cure to form an integral film when exposed to a source of radiation. These properties make it possible to provide substantially continuous coating despite the fact that the film is very thin. The cured film exhibits excellent adhesion and is resistant to chemical attack by organic solvents and inorganic salts.

Because of their reactivity, physical properties and the properties of cured films formed from such components, polyfunctional acrylates are particularly useful monomeric materials. The general formula for such polyfunctional acr-

ylates is:

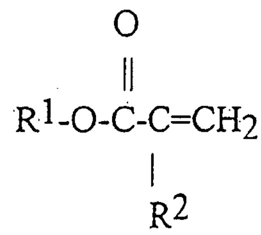

wherein:

$R^1$ is an aliphatic, alicyclic or mixed aliphatic-alicyclic radical; and
$R^2$ is a hydrogen, methyl, ethyl, propyl, butyl or pentyl.

Such polyfunctional acrylates may also be used in combination with various monacrylates, such as those having the formula:

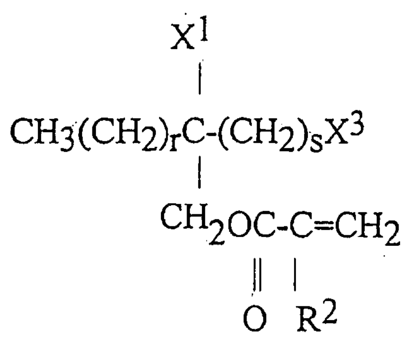

wherein:

$R^2$, is as defined above;
$X^1$ is H, epoxy, 1,6-hexanediol, tripropyleneglycol or urethane;
r, s are 1-18; and
$X^3$ is CN or $COOR^3$.

Diacrylates of the formula below are particularly preferred:

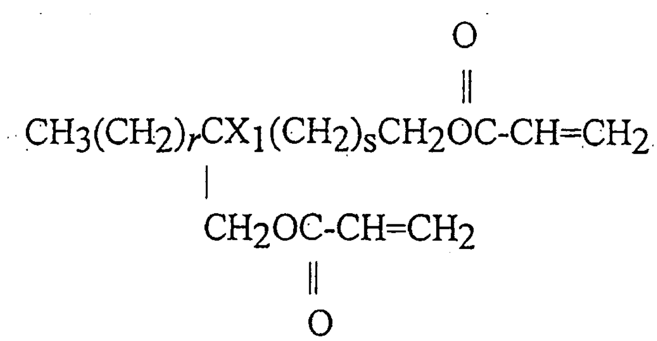

wherein:

$X_1$, r and s are as defined above.

Curing is accomplished by opening the double bonds of the reactant molecules. This can be accomplished by means of an energy source such as apparatus which emits imfrared, electrons or ultraviolet radiation.

FIG. 6 illustrates the process for applying an acrylate material. An acrylate monomer **100** is directed through a dielectric evaporator **102** and then through an ultrasonic atomizer **104** and into a vacuum chamber **106**. The monomer droplets are atomized ultrasonically and the droplets vaporized where they condense on the rotating tube or film that is loaded on a drum **108**.

The condensed monomer liquid subsequently is radiation cured by means of an electron beam gun **110**.

The inorganic material is an oxide material and may be formed by radio frequency discharge, direct or dual ion beam deposition, sputtering or plasma chemical vapor deposition, as described in U.S. -A- 4,698,256, 4,809,876, 4,992,298 and 5,055,318.

For example, a method of depositing an oxide material is provided by establishing a glow discharge plasma in a previously evacuated chamber. The plasma is derived from one or more gas stream components, and preferably is derived from the gas stream itself. The article is positioned in the plasma, preferably adjacent the confined plasma, and the gas stream is controllably flowed into the plasma. An oxide based film is deposited to a desired thickness. The thickness of the oxide material is 10 nm to 1000 nm (100 Angstroms (Å) to 10,000 Å). A thickness of less than 50 nm (500 Å) may not provide sufficient barrier and a thickness of greater than 500 nm (5,000 Å) may crack, thus decreasing the effective barrier. Most preferably, the thickness of the oxide material is 100 nm to 300 nm (1,000 Å to about 3,000 Å).

Another method of depositing an oxide material is by confining a plasma with magnets. Preferably, the magnetically enhanced method for depositing a silicon oxide based film on a substrate is conducted in a previously evacuated chamber of glow discharge from a gas stream. The gas stream preferably comprises at least two components: a volatilized organosilicon component, an oxidizer component such as oxygen, nitrous oxide, carbon dioxide or air and an optionally inert gas component.

Examples of suitable organosilicon compounds useful for the gas stream in the plasma deposition methods are liquid or gas at about ambient temperature and when volatilized have a boiling point about 0°C to about 150°C and include dimethysilane, trimethylsilane, diethylsilane, propylsilane, phenylsilane; hexamethyldisilane, 1,1,2,2-tetramethyldisilane, bis (trimethylsilane)methane, bis (dimethylsilyl) methane, hexamethyldisiloxane, vinyl trimethoxy silane, vinyl triethyoxysilane, ethylmethoxysilane, ethyltrimethoxysilane, divinyltetramethyldisiloxane, hexamethyldsilazane divinylhexamethyltrisiloxane, trivinylpentamethyltrisiloxazane, tetraethoxysilane and tetramethoxysilane.

Among the preferred organosilicons are 1,1,3,3-tetramethyldisiloxane, trimethylsilane, hexamethyldisiloxane, vinyltrimethylsilane, methyltrimethoxysilane, vinyltrimethoxysilane and hexamethyldisilazane. These preferred organosilicon compounds have boiling points of 71°C, 55.5°C, 102°C, 123°C and 127°C respectively.

The optional inert gas of the gas stream preferably is helium, argon or nitrogen.

The volatilized organosilicon component is preferably admixed with the oxygen component and the inert gas component before being flowed into the chamber. The quantities of these gases being so admixed are controlled by flow controllers so as to adjustably control the flow rate ratio of the gas stream components

Various optical methods known in the art may be used to determine the thickness of the deposited film while in the deposition chamber, or the film thickness can be determined after the article is removed from the deposition chamber.

The deposition method of the present invention is preferably practiced at relatively high power and quite low pressure. A pressure less than 66.7Pa (500 millitorr (mTorr)) should be maintained during the deposition, and preferably the chamber is at a pressure between 5.7 to 65.3Pa (43 to 490 millitorr) during the deposition of film. Low system pressure results in lower deposition rates whereas higher system pressure provides faster deposition rates. When the plastic article to be coated is heat sensitive, a higher system pressure may be used to minimize the amount of heat the substrate is exposed to during deposition because high substrate temperatures are to be avoided for low Tg polymers such as polypropylene and PET (Tg is -10°C and 68°C respectively).

The substrate is electrically isolated from the deposition system (except for electrical contact with the plasma) and is at a temperature of less than about 80°C during the depositing. That is, the substrate is not deliberately heated.

Referring to FIG. 7, the system for depositing a silicon oxide material comprises an enclosed reaction chamber **170** in which a plasma is formed and in which a substrate or tube **171**, is placed for depositing a thin film of material on a sample holder **172**. The substrate can be any vacuum compatible material, such as plastic. One or more gases are supplied to the reaction chamber by a gas supply system **173**. An electric field is created by a power supply **174**.

The reaction chamber can be of an appropriate type to perform any of the plasma-enhanced chemical vapor deposition (PECVD) or plasma polymerization process. Furthermore, the reaction chamber may be modified so that one or more articles may be coated with an oxide layer simultaneously within the chamber.

The pressure of the chamber is controlled by a mechanical pump **188** connected to chamber **170** by a valve **190**.

The tube to be coated is first loaded into chamber **170** in sample holder **172**. The pressure of the chamber is reduced to 0.7 Pa (5m Torr) by mechanical pump **188**. The operating pressure of the chamber is 12.0 to 18.7 Pa (90 to 140 mTorr for a PECVD or plasma polymerization process and is achieved by flowing the process gases, oxygen and trimethyl silane, into the chamber through monomer inlet **176**.

The thin film is deposited on the outer surface of the tube and has a desired uniform thickness or the deposition process may be interrupted periodically to minimize heating of the substrate and/or electrodes and/or physically remove particulate matter from the articles.

Magnets **196** and **198** are positioned behind electrode **200** to create an appropriate combination of magnetic and electrical fields in the plasma region around the tube.

The system is suitable for low frequency operation. An example frequency is 40kHz. However, there can be some advantages from operating at a much high frequency, such as in the radio frequency range of several megahertz.

The oxide materials or blends thereof used in accordance with this disclosure, may contain conventional additives and ingredients which do not adversely affect the properties of articles made therefrom.

An optional additional material may be formed on the non-ideal barrier coating sequence composition by dip-coating, roll-coating or spraying an aqueous emulsion of the polyvinylidene chloride or homo or co-polymers, followed by air drying.

The optional additional material may preferably be vinylidene chloride-acrylonitrile-methyl methacrylate-methyl acrylate-acrylic acid copolymers, thermosetting epoxy coatings, parylene polymers, or polyesters.

Preferably, the optional additional material is a parylene polymer. Parylene is the generic name for members of the polymer series developed by Union Carbide Corporation. The base member of the series, called parylene N, is poly-p- xylene, a linear, crystalline material:

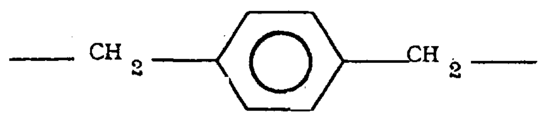

Parylene C, a second member of the parylene series is produced from the same monomer as parylene N and modified by the substitution of a chlorine atom for one other aromatic hydrogens:

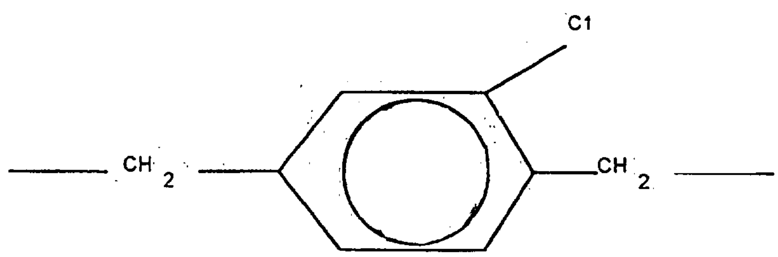

Parylene D, the third member of the parylene series is produced from the same monomer as parylene N and modified by the substitution of the chlorine atom for two of the aromatic hydrogens:

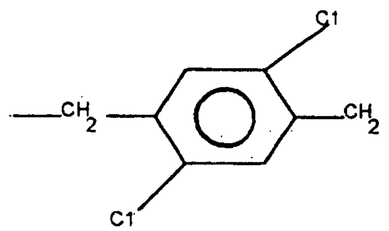

Most preferably, the polymer material is a vinylidene chloride-methyl methacrylate-methacrylate acrylic acid polymer (PVDC). This polymer is available as DARAN® 8600-C (trademark of W.R. Grace and Co.) sold by GRACE, Organic Chemicals Division, Lexington, Mass.

The optional additional material may be a parylene polymer. The optional additional material may be processed similar to vacuum metallizing, as described in U.S. - A - 3,342,754 and 3,300,332.

Alternatively, the optional additional material may be vinylidene chloride-acrylonitrile-methyl methacrylate-methyl acrylate-acid acrylic polymer. This material is applied by dip-coating, roll-coating or spraying an aqueous emulsion of the polymer, followed by air drying of the coating, as described in U.S. - A - 5,093,194 and 4,497,859.

A variety of substrates can be coated with the non-ideal barrier coating sequence composition by the process of the present invention. Such substrates include, but are not limited to, container, bottles, jars, and tubes.

A plastic blood collection tube coated with the non-ideal barrier coating sequence composition will not interfere with testing and analysis that is typically performed on blood in a tube. Such tests include but are nof limited to, routine chemical analysis, biological ineptness, hematology, blood chemistry, blood typing, toxicology analysis or therapeutic drug monitoring and other clinical tests involving body fluids. Furthermore, a plastic blood collection tube coated with the non-ideal barrier coating sequence composition is capable of being subjected to automated machinery such as centrifuges and may be exposed to certain levels of radiation in the sterilization process with substantially no change in optical or mechanical and functional properties.

A plastic blood collection tube coated with the non-ideal barrier coating sequence composition is able to maintain 90% original draw volume over a period of one year. Draw volume retention depends on the existence of a particle vacuum, or reduced pressure, inside the tube. The draw volume changes in direct proportion to the change in vacuum (pressure). Therefore, draw volume retention is dependent on good vacuum retention. A plastic tube coated with the non-ideal barrier coating sequence composition substantially prevents gas permeation through the tube material so as to maintain and enhance the vacuum retention and draw volume retention of the tube. Plastic tubes without the non-ideal barrier coating sequence composition coating of the present invention may maintain about 90% draw volume for about 2 years.

It will be understood that it makes no difference whether the plastic composite container is evacuated or not evacuated in accordance with this invention. The presence of the non-ideal barrier sequence coating composition on the outer surface of the container has the effect of maintaining the general integrity of the container holding a sample so that it may be properly disposed of without any contamination to the user. Notable is the clarity of the non-ideal barrier coating sequence composition and its abrasion and scratch resistance.

The non-ideal barrier coating sequence composition used in accordance with this disclosure, may contain conventional additives and ingredients which do not adversely affect the properties of articles made therefrom.

The following examples are not limited to any specific embodiment of the invention, but are only exemplary. The examples illustrate the production of the non-ideal barrier coating sequence mentioned in the claims and its individual components.

## EXAMPLE 1

## METHOD FOR APPLYING ACRYLATE TO A SUBSTRATE

An acrylate coating was applied to tubes and films (substrates) of various thickness in a chamber wherein Tripropylene Glycol Diacrylate (TPGDA) was fed into the evaporator and was flash vaporized at about 343°C onto the substrate in the chamber and condensed. The condensed monomer film was then E-beam cured by an electron beam gun.

## EXAMPLE 2

## METHOD FOR APPLYING $SIO_X$ TO A SUBSTRATE

The substrate from Example 1 above was then attached to a holder which fits midway between the electrodes in a vacuum chamber. The chamber was closed and a mechanical pump was used to achieve a base pressure of 6.7Pa (50 mTorr).

The electrode configuration was internally capacitively coupled with permanent magnets on the backside of the titanium electrodes. This special configuration provided the ability to confine the glow between the electrodes because of the increase in collision probability between electrons and reacting gas molecules. The net result of applying a magnetic field is similar to increasing the power applied to the electrodes, but without the disadvantages of higher bombardment energies and increased substrate heating. The use of magnetron discharge allows operation in the low pressure region and a substantial increase in polymer deposition rate.

The monomer which consists of a mixture of trimethylsilane (TMS) and oxygen was introduced through stainless steel tubing near the electrodes. The gases were mixed in the monomer inlet line before introduction into the chamber. Flow rates were manually controlled by stainless steel metering valves. A power supply operating at an audio

frequency of 40 kHz was used to supply power to the electrodes. The system parameters used for thin film deposition of plasma polymerized $TMS/O_2$ on the substrate were as follows:

| | |
|---|---|
| Surface Pretreatment | TMS Flow = 0 sccm<br>Base Pressure = (50 mTorr) 6.7Pa<br>Oxygen Flow = 10 sccm<br>System Pressure = (140 mTorr) 18.7 Pa<br>Power = 50 watts<br>Time = 2 minutes |
| Oxide Depositio | TMS Flow = 0.75 - 1.0 sccm<br>Oxygen Flow = 2.5 - 3.0 sccm<br>System Pressure = (90 - 100 mTorr) 12.0-13.3 Pa<br>Power = 30 watts<br>Deposition Time = 5 minutes |

After the thin film was deposited, the chamber was allowed to cool. The chamber was then opened, and the substrate was removed.

## EXAMPLE 3

## METHOD FOR APPLYING A NON-IDEAL BARRIER COATING SEQUENCE COMPOSITION TO A SUBSTRATE

A non-ideal barrier coating sequence composition was applied to a substrate by repeating Examples 1 and 2 above from 1 to 20 times

## EXAMPLE 4

## METHOD FOR APPLYING ACRYLATE TO A SUBSTRATE

An acrylate coating was applied to tubes and films (substrates) in a chamber wherein a 60:40 mixture of isobornyl acrylate: epoxydiacrylate (IBA:EDA) was fed into the evaporator and flash vaporized at about 343°C onto the substrate in the chamber and condensed. The condensed monomer film was then UV cured by an actinic light source of 365 nm.

## EXAMPLE 5

## METHOD FOR APPLYING $SIO_X$ TO A SUBSTRATE

The substrate from Example 4 was then attached to a holder which fits midway between the electrodes in a vacuum chamber. The chamber was closed and a mechanical pump was used to achieve a base pressure of 6.7Pa (50 mTorr).

The electrode configuration was internally capacitively coupled with permanent magnets on the backside of the titanium electrodes. The special configuration provided the ability to confine the glow between the electrodes because of the increase in collision probability between electrons and reacting gas molecules. The net result of applying a magnetic field is similar to increasing the power applied to the electrodes, but without the disadvantages of higher bombardment energies and increased substrate heating. The use of magnetron discharge allows operation in the low pressure region and a substantial increase in polymer deposition rate.

The monomer which consists of a mixture of trimethylsilane (TMS) and oxygen was introduced through stainless steel tubing near the electrodes. The gases were mixed in the monomer inlet line before introduction into the chamber. Flow rates were manually controlled by stainless steel metering valves. A power supply operating at an audio frequency of 40 kHz was used to supply power to the electrodes. The system parameters used for thin film deposition of plasma polymerized $TMS/O_2$ on the substrate were as follows:

| | |
|---|---|
| Surface Pretreatment | TMS Flow = 0 sccm<br>Base Pressure = (50 mTorr) 6.7Pa<br>Oxygen Flow = 10 sccm<br>System Pressure = (140 mTorr) 18.7Pa<br>Power = 50 watts<br>Time = 2 minutes |
| Oxide Deposition | TMS Flow = 0.75 - 1.0 sccm<br>Oxygen Flow = 2.5 - 3.0 sccm<br>System Pressure = (90 - 100 mTorr) 12.0-13.3Pa<br>Power = 30 watts<br>Deposition Time = 5 minutes |

After the thin film was deposited, the chamber was allowed to cool. The chamber was then opened, and the substrate was removed.

**EXAMPLE 6**

**METHOD FOR APPLYING A NON-IDEAL BARRIER COATING SEQUENCE COMPOSITION TO A SUBSTRATE**

A non-ideal barrier coating sequence composition was applied to a substrate by repeating Examples 4 and 5 above from 1 to 20 times

**EXAMPLE 7**

**BEHAVIOR CHARACTERISTICS OF NON-IDEAL COMPOSITE COMPOSITIONS**

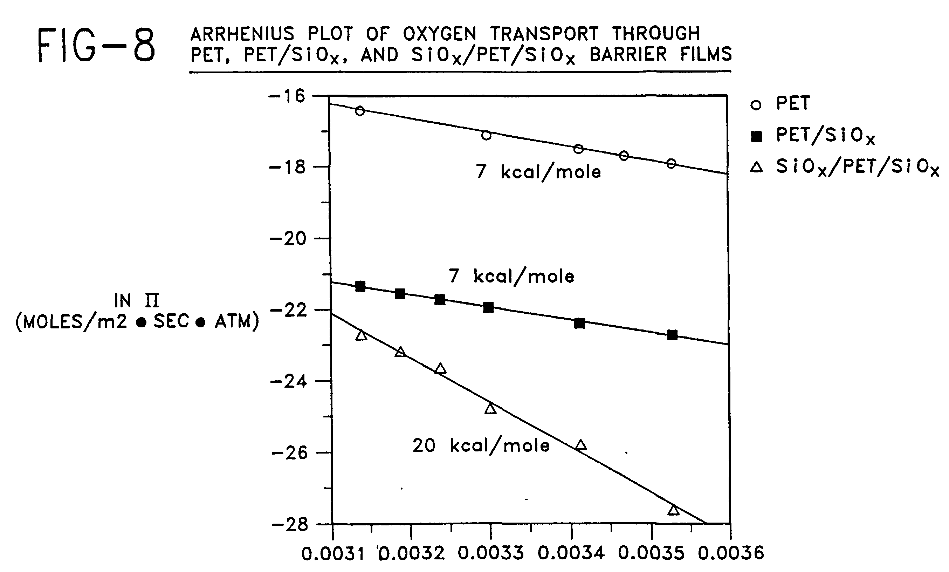

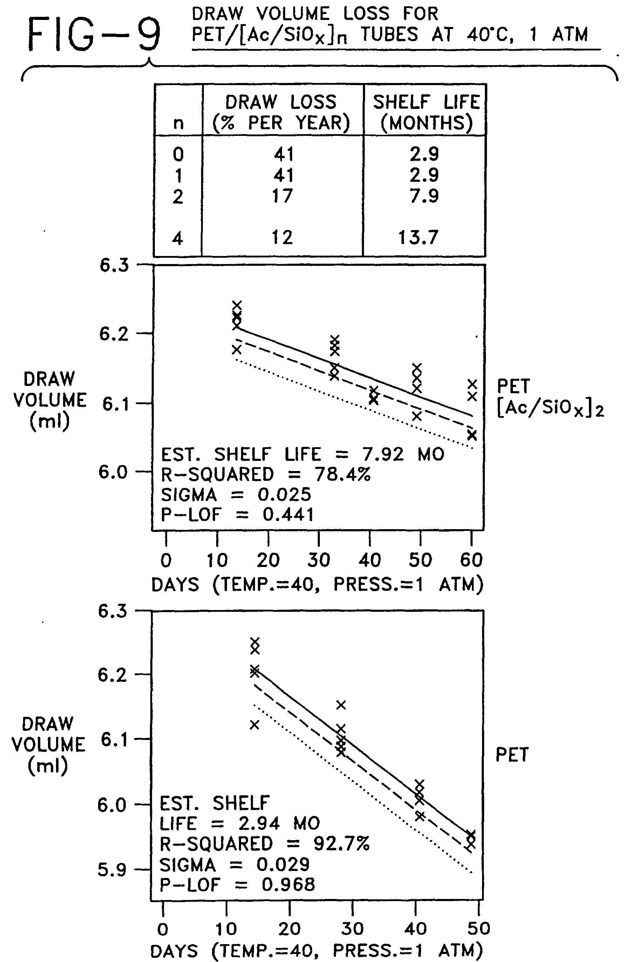

Various different substrates were prepared in accordance with Examples 1-6 above and then the following characteristics and properties were evaluated and the results are reported in Tables 1, 2 and 3 and Figures 8 and 9.

(i) Laminate equation analysis:

When two or more different barrier films are stacked, the permeation of small molecules through the multilayer laminate is generally described by the laminate equation:

$$(\Pi_{12}) = (\Pi_1^{-1} + \Pi_2^{-1})^{-1}$$

or

$$\Pi = (\sum_n [\Pi^{-1}])^{-1}$$

where n is the number of layers of different materials deposited as in Example 6 above, $\Pi_1$, is the permeation rate through component layer 1, $\Pi_2$ is the permeation rate through component layer 2, and $\Pi_{12}$ is the permeation rate through the laminate 'of components 1 and 2. The transport rate of a permeant through a laminate barrier system is therefore dependent upon the transport rate of that permeant through each of the laminate's components. When the permeance of the individual components is known, the permeance of the total layer laminate of those components can be calculated and predicted.

The permeance of oxygen or water through such systems can be obtained at defined temperature and driving pressure by use of a MOCON OX-Tran 2000, MOCON T-1000 or MOCON Permatran device.

(ii) Arrhenius relationship analysis

When the transmission rate of a permeant, such as oxygen or water, through a barrier structure is obtained at several different temperatures, the thermodynamic energy necessary to transport the permeant completely through the barrier structure is obtained by the Arrhenius equation:

$$\ln Q = \ln Q_0 - \Delta G/RT$$

where ΔG is the energy necessary to move one mole or perment molecules through the barrier structure in cal/mole, R is the gas constant in cal/mole - degree, T is temperature in degrees Kelvin, Q is the permeant transmission rate and $Q_o$ is a constant unique to the structure. In practice, the transmission rate Q for oxygen transport through the barrier structure is the permeance Π, obtained at several temperatures. Then the natural log of the transmission rate obtained at each temperature versus the reciprocal of each temperature is plotted The slope of the resultant linear plot is -ΔG/R, from which ΔG is obtained.

These data are obtained at several defined temperatures, using the same equipment as described above. The resulting permeance data (Π) are then treated by the Arrhenius equation, and ΔG values for the laminate are compared to ΔG values obtained for the components of the laminate. An ideal laminate system has a ΔG equivalent to that of the component with the best barrier characteristics. A non-ideal system has a ΔG greater than that of either component

(iii) Oxygen permeance (OTR):

Film or plaque samples were tested for oxygen permeance (OTR) using a MO CON Ox-TRAN 2/20 (sold by Modem Controls, Inc., 7500 Boone Avenue N., Minneapolis, MN 55428). A single side of the film sample was exposed to 1 atm of 100% oxygen atmosphere. Oxygen permeating through the sample film was entrained in a nitrogen carrier gas stream on the opposite side of the film, and detected by a coulmetric sensor. An electrical signal was produce in proportion to the amount of oxygen permeating through the sample. Samples were tested at 30, 35, 40 and 45°C and 0% relative humidity (R.H.). Samples were conditioned for 1 to 20 hours prior to determining oxygen permeance. The results obtained for polycarbonate (PC) and polyethylene terephthalate (PET) are reported in Tables 1 and 3.

Tube samples were tested for oxygen permeance (OTR) using a MOCON $O_x$-TRAN 1,000 (sold by Modern Controls, Inc., 7500 Boone Avenue N., Minneapolis, MN 55428). A package adapter was used for mounting the tubes in a manner that allowed the outside of the tube to be immersed in a 100% $O_2$ atmosphere while the inside of tube is flushed with a nitrogen carrier gas. The tubes were then tested at 30°C and 50% R.H. The tubes were allowed to equilibrate for 2-14 days before a steady state permeability is determined. The results for polyester based blood collection tubes is reported in Table 4, and the results for polypropylene based blood collection tubes is reported in Table 5.

(iv) Water Vapor Transmission Rate (WVTR):

a. Tubes: Tubes were filled with a 2 ml of distilled water, closed with a rubber stopper, and placed into an oven at 40°C, 50% R.H. The tubes were then weighed once a week for 4 months. The water vapor transmission rates were then calculated based on the equilibrium water loss per day. Examples of PET based blood collection tubes are reported in Table 4:

b. Films: Films were placed into the Permeatran W-600 instrument at 40°C. A single side. of the film was exposed to 100% relative humidity (RH). Water vapor permeating through the sample film was introduced into an IR sensor by a nitrogen carrier gas. An electrical signal was produced in proportion to the amount of water vapor permeating through the sample. Data was collected at 25, 30 and 35°C for use in the Arrhenius equation, $\ln Q = \ln Q - \Delta G/RT$.

**A. <u>Non-Ideal Barrier Coating Sequence Compositions on Films</u>**

Samples 1-4 of Table 1 and Figure 8 illustrate that coating both sides of a PET film results in an unexpected decrease in the oxygen transmission rate that the laminate equation does not predict. The measured and predicted oxygen transport rate of films coated with $SiO_x$ were compared to the separately measured oxygen transport rate of PET and $SiO_x$. The results are reported in Figure 8 and Table 1. This occurs despite the fact that the PET/$SiO_x$ sample behaved as a predictable laminate.

Figure 8 is the Arrhenius equation plot of the natural log of the transmission rates versus the reciprocal of the

temperature at which each measurement was taken for Samples 1, 3 and 4. From Figure 8 ΔG, or the energy required to transport one mole of oxygen through $SiO_x$/PET/$SiO_x$, Sample 4, is 20± 1 kcal/mole as compared to the ΔG of 7 ± 2kcal/mole for PET, Sample 1 and PET/$SiO_x$, Sample 3.

This result shows that the non-ideal barrier coating composition of $SiO_x$/PET/$SiO_x$ results in the expenditure of more thermal energy, ΔG than the individual components of the composition. This is consistent with the relationship of the non-ideal barrier sequence coating composition of the present invention, $\Delta G_T > \Delta G_A, \Delta G_B$, where T is Sample 4 and A and B are Samples 1 and 2 respectively.

Samples 1, 2 and 3 illustrate that PET/$SiO_x$, Sample 3 acts only as a laminate of PET and $SiO_x$, which can be predicted, wherein $SiO_x$/PET/$SiO_x$, Sample 4 exhibits non-ideal permeance results. Therefore this illustrates that the barrier performance of Sample 4 as a whole is independent of the properties of its constituents, with respect to oxygen permeation.

In addition, Table 2 illustrates the unexpected increase in water vapor barrier performance of Sample 9 ($SiO_x$/PET/$SiO_x$). The observed water vapor transmission rate through the structure is 30 to 50 times lower than the value predicted from laminate theory, based on the transmission rates through Sample 5 (PET film), Sample 6 ($SiO_x$) and Sample 7 (PET/$SiO_x$).

Samples 10-18 of Table 3 illustrate that the oxygen permeance of PC/$SiO_x$/acrylate/$SiO_x$ (Sample 16) is $1.1 \cdot 10^{-10}$ moles/$m^2 \cdot s \cdot 1.013 \cdot 10^5$ Pa ($1.1 \times 10^{-10}$ moles/$m^2$·sec·atm) as compared to the $16.4 \cdot 10^{-10}$ moles/$m^2 \cdot s \cdot 1.013 \cdot 10^5$ Pa ($16.4 \times 10^{-10}$ moles/$m^2$·sec·atm) that is predicted (Sample 18) with the laminate equation.

In addition, Samples 10-16 also illustrate that the water permeance of PC/$SiO_x$/acrylate/$SiO_x$ (Sample 16) is $0.9 \cdot 10^{-10}$ moles/$m^2 \cdot s \cdot 1.013 \cdot 10^5$ Pa ($0.9 \times 10^{-10}$ moles/$m^2$·sec·atm) as compared to the $4.9 \cdot 10^{-10}$ moles/$m^2 \cdot s \cdot 1.013 \cdot 10^5$ Pa ($4.9 \times 10^{-10}$ moles/$m^2$·sec·atm) predicted (Sample 18) with the laminate equation. The result of Sample 16 shows that the barrier performance of the non-ideal barrier coating sequence composition of PC/$SiO_x$/acrylate/$SiO_x$ as a whole is greater that the sum of each individual material in the whole composition, with respect to both oxygen and water.

Also noted is that Sample 15 (PC/acrylate/$SiO_x$) (measured) behaved as a single laminate which indicates that Sample 16 (PC/$SiO_x$/acrylate/$SiO_x$) should have also exhibited simple laminate characteristics. However, as shown in Table 3, Sample 16, (PC/$SiO_x$/acrylate/$SiO_x$) did not exhibit simple laminate characteristics. Therefore, it can be concluded that Sample 16 follows the modified laminate equation for a non-ideal barrier coating sequence composition of

$$(\Pi_{PC/SiO_x/acrylate/SiO_x})^{-1} < (\Pi_{PC})^{-1} + (\Pi_{acrylate})^{-1} + 2\,(\Pi_{SiO_x})^{-1}$$

where $(\Pi_{PC})^{-1}$ is Sample 10, $(\Pi_{acrylate})^{-1}$ is Sample 12, and $(\Pi SiO_x)^{-1}$ is Sample 11.

Consistent with the laminate characteristics of Sample 16 (PC/$SiO_x$/acrylate/$SiO_x$) are the ΔG values (energy necessary to transport oxygen or water through the sample) observed for this sample. The ΔG value for oxygen transport through Sample 16, (PC/$SiO_x$/acrylate/$SiO_x$), is 37.7 kg/mole (9 kcal/mole) higher than the energy necessary to transport one mole of oxygen through the PC, PC/$SiO_x$ or PC/acrylate/$SiO_x$ systems (Samples 10, 14 and 15). This result shows that the non-ideal barrier coating sequence composition results in the expenditure of more thermal energy, ΔG, than any of the components of the composition. This is consistent with the relationship of $\Delta G_T > G_A, \Delta G_B, \Delta G_C$ where T is Sample 16 and A, B and C are Samples 10, 14 or 15, respectively.

The ΔG value for water transport through Sample 16 follow the same non-ideal characteristics as reported for oxygen transport. The Δ The ΔG values for transport through PC/$SiO_x$ and PC/acrylate/$SiO_x$, are 3-4 kcal/mole higher than the ΔG of transport through PC alone. However, the water transmission rate through the PC/$SiO_x$/acrylate/$SiO_x$ structure is not predicted by the laminate equation as compared to Sample 18. These non-ideal results show an unexpected enhancements of water vapor barrier and that $\Delta G_T > \Delta G_A, \Delta G_B, \Delta G_C$ where T is Sample 16 and A, B and C are Samples 10, 14 and 15 respectively.

## B. Non-Ideal Barrier Coating Sequence Compositions on Tubes

In addition, an unexpected improvement in barrier properties for PET and PP coated tubes was observed. Tables 4 and 5 summarize the barrier characteristics of PET and PP tubes coated with (acrylate/$SiO_x)_n$.

Samples 19-27 (Table 5) illustrate that PET tubes coated with more than 1 sequence of acrylate/$SiO_x$ exhibit non-ideal behavior with respect to oxygen and water vapor permeance, wherein experimental water vapor and oxygen transmission rates are significantly lower than theoretically predicted based on the laminate equation.

Samples 25-27 follow the modified laminate equation for a non-ideal barrier coating sequence composition as follows:

$$\Pi_{PET\ (acrylate/SiO_x)n} < ((\Pi_{PET})_1^{-1} + n(\Pi_{acrylate})^{-1} + n(\Pi_{SiOx})^{-1})^{-}$$

where PET (acrylate/$SiO_x$)$_n$ is Samples 22-25, PET is Sample 19 and acrylate is Sample 23 and $SiO_X$ is Sample 22.

It is observed that the deposition of a single sequence of acrylate/$SiO_x$ does not improve 'water barrier properties of the PET tube, however, two sequences of acrylate/$SiO_x$ (Sample 25) result in decreasing water vapor transmission rates by a factor of 17.5 compared to the PET control (Sample 19). As a result of a non-ideal barrier behavior, tubes coated with 4 and 6 sequences of acrylate/$SiO_x$ have remarkably low oxygen and water vapor permeance, superior to any transparent barrier coating on a plastic substrate reported in the literature as shown in Table 6.

In addition, Samples 28-33 (Table 5) illustrate non-ideal behavior of the PP tubes coated with 2 sequences of acrylate/$SiO_x$ The measured oxygen permeance of PP/(acrylate/$SiO_x$)$_2$ sequence (Sample 33) is 11.5 x $10^{-10}$ moles/$m^2$·sec·atm, compared to 496 x $10^{-10}$moles/$m^2$·sec·atm predicted with the laminate equation. Therefore, it can be concluded that Sample-31 follows the modified laminate equation for a non-ideal barrier coating sequence composition of

$$\Pi_{PP}/(acrylate/SiO_x)_2 < (\Pi_{PP})^{-1} + 2(\Pi_{acrylate})^{-1} + 2(\Pi_{SiOx})^{-1}$$

where PP/(acrylate/$SiO_x$)$_2$ is Sample 33, PP is Sample 28, acrylate is Sample 32 and SiOx is Sample 30.

TABLE I

BARRIER CHARACTERISTICS OF FILMS

| | | Oxygen Transport Rate | | | | Oxygen Permeance | | |
|---|---|---|---|---|---|---|---|---|
| Sample No. | Sample Description | Q measured $10^{-10}$ mole/m²·s·1.013·10⁵ Pa ($10^{-10}$ mole/m²·sec·atm)@30°C | Q predicted (6) $10^{-10}$ mole/m²·s·1.013·10⁵ Pa ($10^{-10}$ mole/m²·sec·atm)@30°C | ΔG 4.186kJ/mole kcal/mole @30°C | Barrier Type | $\Pi_{O2}$@35°C $10^{-10}$ mole/m²·s·1.013·10⁵ Pa ($10^{-10}$ mole/m²·sec·atm) | $\Pi_{O2}$@40°C $10^{-10}$ mole/m²·s·1.013·10⁵ Pa ($10^{-10}$ mole/m²·sec·atm) | $\Pi_{O2}$@45°C $10^{-10}$ mole/m²·s·1.013·10⁵ Pa ($10^{-10}$ mole/m²·sec·atm) |
| 1 | PET film | 403 | -- | 7±2 | Monolithic(1) | | | |
| 2 | $SiO_x$ | 2.73 | -- | | Monolithic(1) | | | |
| 3 | PET/$SiO_x$ | 2.71 | 2.71 | 7±2 | Laminate(2) | 3.59 (4) | 4.28 (4) | 5.17 (4) |
| 4 | $SiO_x$/PET/$SiO_x$ | 0.56 | 1.35 | 20±1 | Composite(3) | 0.53 (5) | 0.91 (5) | 1.36 (5) |

(1) Monolithic = single component barrier

(2) Laminate = two component barrier with ideal characteristics

(3) Composite = two component barrier with non-ideal characteristics

(4) $\Pi^{-1} = \Pi^{-1}_{PET} + \Pi^{-1}_{SiOx}$

(5) $\Pi^{-1} < \Pi^{-1}_{PET} + 2\Pi^{-1}_{SiOx}$ (composite)

TABLE 2

BARRIER CHARACTERISTICS OF FILMS

| Sample No. | Sample Description | Water Vapor Transmission Rates ($10^{-7}$ Mole/$m^2$, sec) | | | |
|---|---|---|---|---|---|
| | | 25° | 30° | 35° | 40° |
| 5 | PET film | 153 | 212 | 318 | 424 |
| 6 | $SiO_x$ | 14.6 | 21.1 | 30.2 | 75.4 |
| 7 | PET/$SiO_x$ | 13.31 | 19.16 | 27.58 | 63.98 |
| 8 | $SiO_x$/PET/$SiO_x$ (observed) | 0.239 | 0.289 | 0.450 | 0.64 |
| 9 | $SiO_x$/PET/$SiO_x$ (1) | 7.0 | 10.0 | 14.4 | 34.6 |

(1) predicted from laminate equation: $(\Pi_{SiO_x/PET/SiO_x})^{-1} = (\Pi_{PET})^{-1} + 2(\Pi_{SiO_x})^{-1}$

TABLE 3

BARRIER CHARACTERISTICS OF FILMS

| Sample No. | Sample Description | $O_2$ transmission rate $10^{-10}$ mole/m²·s·1.013·$10^5$ Pa ($10^{-10}$ mole/m²·sec·atm) | $H_2O$ vapor transmission rate $10^{-10}$ mole/m²·s·1.013·$10^5$ Pa ($10^{-10}$ mole/m²·sec·atm) | $\Delta G_{O_2}$ 4.186 kJ/mole (kcal/mole) | $\Delta G_{H_2O}$ 4.186 kJ/mole (kcal/mole) |
|---|---|---|---|---|---|
| 10 | PC film | 7233.8 | 252.7 | 4.4 ± 0.16 | 9.8 ± 0.29 |
| 11 | $SiO_x$ | 31.2 | 9.7 | | |
| 12 | acrylate | 4818.6 | >15,000 | | |
| 13 | PC/acrylate | 2893.5 | 249.5 | | |
| 14 | PC/$SiO_x$ | 31.0 | 9.3 | 4.2 ± 0.20 | 13.1 ± 1.10 |
| 15 | PC/acrylate/$SiO_x$(measured) | 34.9 | 9.7 | 4.6 ± 0.30 | 12.8 ± 0.9 |
| 16 | PC/$SiO_x$/acrylate/$SiO_x$ (measured) | 1.1 | 0.9 | 13.3±2.0 | 14.6 ± 1.71 |
| 17 | PC/acrylate/$SiO_x$ (predicted) (1) | 33.7 | 9.7 | | |
| 18 | PC/$SiO_x$/acrylate/$SiO_x$ (predicted) (2) | 16.4 | 4.9 | | |

(1) Predicted by: $\Pi^{-1} = \Pi^{-1}_{pc} + \Pi^{-1}_{acrylate} + \Pi^{-1}_{SiOx}$ (sample 17)

(2) Predicted by: $\Pi^{-1} = \Pi^{-1}_{pc} + \Pi^{-1}_{acrylate} + 2\Pi^{-1}_{SiOx}$ (sample 18)

TABLE 4

BARRIER CHARACTERISTICS OF TUBES

| | | WATER VAPOR TRANSMISSION | | OXYGEN PERMEANCE | | |
|---|---|---|---|---|---|---|
| Sample No. | Sample Description | Measured Water Vapor Transmission Rate ($10^{-10}$ moles/$m^2$·sec @ 40°C, 50% R.H.) | Theoretical Water Vapor Transmission Rate ($10^{-10}$ moles/$m^2$·sec @ 40°C, 50% R.H.) | Measured Oxygen Transmission Rate $10^{-10}$ mole/$m^2$·s·$1.013 \cdot 10^5$ Pa ($10^{-10}$ mole/$m^2$·sec·atm @ 40°C, 0 % R.H. | Theoretical Oxygen Transmission Rate $10^{-10}$ mole/$m^2$·s·$1.013 \cdot 10^5$ Pa ($10^{-10}$ mole/$m^2$·sec·atm @ 40°C, 0 % R.H. | Barrier Type |
| 19 | PET tube, control | 369 | | 67.8 | -- | Monolithic |
| 20 | PET/$SiO_x$ | 370 | | 67.2 | -- | |
| 21 | PET/acrylate | 370 | | 67.4 | -- | |
| 22 | $SiO_x$ | 135,000 | | | | |
| 23 | acrylate | 137,000 | | | | |
| 24 | PET (acrylate/$SiO_x$)1 (1) | 370 | 370 | 66.6 | 66.8 | Laminate |
| 25 | PET (acrylate/$SiO_x$)2 (2) | 101 | 370 | 3.8 | 66.6 | Laminate |
| 26 | PET (acrylate/$SiO_x$)4 (3) | 64.5 | 370 | 1.9 | 65.5 | Laminate |
| 27 | PET (acrylate/$SiO_x$)6 (4) | 24.7 | 370 | -- | -- | Laminate |

(1) 1 sequence of acrylate/$SiO_x$

(2) 2 sequences of acrylate/$SiO_x$

(3) 4 sequences of acrylate/$SiO_x$

(4) 6 sequences of acrylate/$SiO_x$

TABLE 5

BARRIER CHARACTERISTICS OF TUBES

| Sample No. | Sample Description | Measured Oxygen Transmission Rate $10^{-10}$ moles/$m^2 \cdot s \cdot 1.013 \cdot 10^5$ Pa ($10^{-10}$ moles/$m^2 \cdot$ sec·atm) | Theoretical Oxygen Transmission Rate $10^{-10}$ moles/$m^2 \cdot s \cdot 1.013 \cdot 10^5$ Pa ($10^{-10}$ moles/$m^2 \cdot$ sec·atm) |
|---|---|---|---|
| 28 | PP tube, control (1) | 1120 | |
| 29 | PP/$SiO_x$ | 1000 | |
| 30 | $SiO_x$ | 9300 | |
| 31 | PP/acrylate | 739.5 | -- |
| 32 | acrylate | 2200 | -- |
| 33 | PP/(acrylate/$SiO_x$)$_2$ (2) | 11.5 | 496 |

(1) 1 sequence of acrylate/$SiO_x$

(2) 2 sequences of acrylate/$SiO_x$

| TABLE 6 |
|---|
| Reference |
| Proc. Int. Conf. Vac. Web. Coat, 5th, 123-127, Italy |
| JP-A-4904169 |
| Proc. Int. Conf. Vac. Web. Coat. 5th, 86-103 |
| JP-A-91-277164 |
| JP-A- 04103757 |
| EP-A-469926 |
| US - A - 3,442,686 |
| US - A - 4,702,963 |
| US - A - 4,557,946 |

**Claims**

1. A plastic container having an open end, a closed end, an inner surface and an outer surface; comprising a non-ideal barrier coating sequence associated over the outer surface of said container composition comprising:

$$\sum_{n} (\text{organic material} + \text{inorganic material})$$

where n = 1-20,

wherein said inorganic material is deposited onto said organic material; and wherein an organic material selected from poly(vinylidenechloride), vinylidene chloride-methyl methacrylate-methacrylate acrylic acid polymer (PVDC), vinylidene chloride-acrylonitrile-methyl methacrylate-methylacrylate-acrylic acid copolymers, thermosetting epoxy materials, parylene polymers or polyesters is disposed over the non-ideal barrier coating sequence.

2. The container of Claim 1, wherein the permeation rate of the lamination of said organic and inorganic materials ($\Pi_{oi}$), is less than the inverse of the sum of the inverse of the permeation rate through said inorganic material ($II_i$) of said sequence and the inverse of the permeation rate through said organic material ($\Pi_o$) of said sequence.

3. The container of Claim 2, wherein said permeation rate of the lamination of said organic and inorganic materials is not an additive effect.

4. The container of Claim 1, wherein the thermal energy ($\Delta G_T$) of said non-ideal barrier coating sequence composition is greater than the thermal energy of said organic component ($\Delta G_A$) and said inorganic component ($\Delta G_B$).

5. The container of Claim 1, wherein said organic material is a highly crossed linked acrylate or acrylic polymer.

6. The container of Claim 1, wherein the inorganic material is a metal oxide.

7. The container of Claim 6, wherein said metal oxide is a silicon oxide based composition or an aluminium oxide based composition.

**8.** The container of Claim 7, wherein said silicon oxide based composition is silicon oxide, $SiO_x$, where x is from 1.0 to 2.5.

**9.** The container of Claim 5, wherein the thickness of said organic material is 0.1 micrometer to 10 micrometer.

**10.** The container of Claim 9, wherein the thickness of said organic material is 0.5 micrometer to 3 micrometer.

**11.** The container of Claim 6, wherein the thickness of said metal oxide its 1 µm to 20 µm (100 to 2,000 Angstroms).

**12.** The container of Claim 11, wherein the thickness of said metal oxide is 5 µm to 10 µm (500 to 1,000 Angstroms).

**13.** The container of Claim 1, wherein said thickness of said poly(vinylidene chloride) is 2 to 15 micrometers.

**14.** The container of Claim 13, wherein said thickness of said poly(vinylidene chloride) is 3 to 5 micrometers.

**15.** The container of Claim 1, wherein said organic material is a polymerized blend of mono-and di-acrylates.

**16.** The container of Claim 1, wherein said organic material comprises polymerized acrylate and said inorganic material comprises silicon oxide.

**17.** The container of Claim 1, wherein said acrylate of said organic material is deposited on said outer surface of said container in a previously evacuated chamber comprising the following steps:

(a) metering a curable monomer component into a heated vaporizer system;
(b) flash vaporizing said component in said chamber;
(c) condensing, vaporizing or atomizing a film of the component onto the outer surface of said container; and
(d) curing said film.

**18.** The container of Claim 17, wherein said inorganic material is deposited in said previously evacuated chamber onto said organic material by the following steps:

(a) vaporizing an organosilicon component and admixing the volatilized organosilicon component with an oxidizer component and optionally an inert gas component to form a gas steam exterior to the chamber;
(b) establishing a glow discharge plasma in the chamber from one or more of the gas stream components;
(c) controllably flowing the gas stream into the plasma while confining at least a portion of the plasma therein; and
(d) depositing a layer of silicon oxide adjacent said first layer.

**19.** The container of Claim 18, wherein said oxidizer component is oxygen nitrous oxide, carbon dioxide, air, or an inert compound.

**20.** The container of Claim 19, wherein the plastic substrate is electrically isolated from the chamber except for contact with the confined plasma.

**21.** The container of Claim 1, wherein said parylene polymer is parylene N, parylene C or parylene D.

**22.** A method of depositing a non-ideal barrier coating sequence composition on the outer surface of a plastic container comprising:

(a) selecting a curable component comprising: i) polyfunctional acrylates, or ii) mixtures of monoacrylates and polyfunctional acrylates;
(b) flash vaporizing said component into said chamber;
(c) condensing an acrylate material of said vaporized component onto the outer surface of said container;
(d) curing said acrylate material;
(e) vaporizing an organosilicon component and admixing a volatilized organdsilicon component with an oxidizer component and optionally an inert gas component to form a gas steam exterior to the chamber;
(f) establishing a glow discharge plasma in the chamber from one or more of the gas stream components;
(g) controllably flowing the gas stream into the plasma while confining at least a portion of the plasma therein;

(h) depositing a material of silicon oxide adjacent said acrylate material;
(i) repeating steps (a) through (d) above, thereby depositing an acrylate material on said silicon oxide material; and
(j) repeating steps (e) through (h) above, thereby depositing a silicon oxide material on said acrylate material.
(k) removing the container from the chamber and disposing an organic material selected from poly(vinylidenechloride), vinylidene chloride-methyl methacrylate-methacrylate acrylic acid polymer (PVDC), vinylidene chloride-acrylonitrile-methyl methacrylate-methylacrylate-acrylic acid copolymers, thermosetting epoxy materials, parylene polymers or polyesters over the non-ideal barrier coating sequence.

**23.** The method of ctaim 22 wherein the organic material of step (k) is formed on the non-ideal barrier coating sequence by dip-coating, roll-coating or spraying.

**24.** The method of Claim 22 in which step (k) consists of dip coating PVDC on said non-ideal barrier coating composition sequence.

**25.** The method of Claim 22 further comprising repeating steps (i) through (j) from 2 to 20 times prior to step (k).

**26.** The method of Claim 22 wherein said acrylate material is pretreated by oxygen plasma.

**Patentansprüche**

**1.** Kunststoffbehälter mit einem offenen Ende, einem geschlossenen Ende, einer inneren Oberfläche und einer äußeren Oberfläche, umfassend:

eine nichtideale Sperrbeschichtungssequenz, die über der äußeren Oberfläche der Behälterzusammensetzung angeordnet ist und Folgendes umfasst:

$$\sum_{n} \text{(organisches Material + anorganisches Material)},$$

wobei n = 1-20;
wobei das anorganische Material auf dem organischen Material abgeschieden ist; und
wobei ein organisches Material, das aus Polyvinylidenchlorid, Vinylidenchlorid-Methylmethacrylat-Methylacrylat-Acrylsäure-Polymer (PVDC), Vinylidenchlorid-Acrylnitril-Methylmethacrylat-Methylacrylat-Acrylsäure-Copolymeren, duroplastischen Epoxymaterialien, Parylenpolymeren oder Polyestern ausgewählt ist, über der nichtidealen Sperrbeschichtungssequenz angeordnet ist.

**2.** Behälter gemäß Anspruch 1, wobei die Permeationsrate der Laminierung aus dem organischen und dem anorganischen Material ($\Pi_{oi}$) kleiner ist als der Kehrwert der Summe aus dem Kehrwert der Permeationsrate durch das anorganische Material ($\Pi_i$) der Sequenz und dem Kehrwert der Permeationsrate durch das organische Material ($\Pi_o$) der Sequenz.

**3.** Behälter gemäß Anspruch 2, wobei die Permeationsrate der Laminierung aus dem organischen und dem anorganischen Material kein additiver Effekt ist.

**4.** Behälter gemäß Anspruch 1, wobei die Wärmeenergie ($\Delta G_T$) der nichtidealen Sperrbeschichtungssequenzzusammensetzung größer ist als die Wärmeenergie der organischen Komponente ($\Delta G_A$) und der anorganischen Komponente ($\Delta G_B$).

**5.** Behälter gemäß Anspruch 1, wobei das organische Material ein hochgradig vernetztes Acrylat- oder Acrylpolymer ist.

**6.** Behälter gemäß Anspruch 1, wobei das anorganische Material ein Metalloxid ist.

**7.** Behälter gemäß Anspruch 6, wobei das Metalloxid eine Zusammensetzung auf Siliciumoxidbasis oder eine Zusammensetzung auf Aluminiumoxidbasis ist.

**8.** Behälter gemäß Anspruch 7, wobei es sich bei der Zusammensetzung auf Siliciumoxidbasis um Siliciumoxid $SiO_x$ handelt, wobei x = 1,0 bis 2,5 beträgt.

**9.** Behälter gemäß Anspruch 5, wobei die Dicke des organischen Materials 0,1 µm bis 10 µm beträgt.

**10.** Behälter gemäß Anspruch 9, wobei die Dicke des organischen Materials 0,5 µm bis 3 µm beträgt.

**11.** Behälter gemäß Anspruch 6, wobei die Dicke des Metalloxids 1 µm bis 20 µm (100 bis 2000 Å) beträgt.

**12.** Behälter gemäß Anspruch 11, wobei die Dicke des Metalloxids 5 µm bis 10 µm (500 bis 1000 Å) beträgt.

**13.** Behälter gemäß Anspruch 1, wobei die Dicke des Polyvinylidenchlorids 2 µm bis 15 µm beträgt.

**14.** Behälter gemäß Anspruch 13, wobei die Dicke des Polyvinylidenchlorids 3 µm bis 5 µm beträgt.

**15.** Behälter gemäß Anspruch 1, wobei das organische Material ein polymerisiertes Gemisch von Mono- und Diacrylaten ist.

**16.** Behälter gemäß Anspruch 1, wobei das organische Material polymerisiertes Acrylat umfasst und das anorganische Material Siliciumoxid umfasst.

**17.** Behälter gemäß Anspruch 1, wobei das Acrylat des organischen Materials in einer zuvor evakuierten Kammer auf der äußeren Oberfläche des Behälters abgeschieden wird, wobei die Abscheidung die folgenden Schritte umfasst:

(a) Zudosieren einer härtbaren Monomerkomponente in ein geheiztes Verdampfersystem;

(b) Schnellverdampfen der Komponente in der Kammer;

(c) Kondensieren, Aufdampfen oder Aufstäuben eines Films aus der Komponente auf die äußere Oberfläche des Behälters; und

(d) Härten des Films.

**18.** Behälter gemäß Anspruch 17, wobei das anorganische Material durch die folgenden Schritte in der zuvor evakuierten Kammer auf dem organischen Material abgeschieden wird:

(a) Verdampfen einer siliciumorganischen Komponente und Vermischen der verflüchtigten siliciumorganischen Komponente mit einer Oxidationsmittelkomponente und gegebenenfalls einer Inertgaskomponente unter Bildung eines Gasdampfs außerhalb der Kammer;

(b) Bilden eines Glimmentladungsplasmas in der Kammer aus einer oder mehreren der Gasstromkomponenten;

(c) gesteuertes Strömenlassen des Gasstroms in das Plasma, wobei wenigstens ein Teil des Plasmas darin eingeschlossen wird; und

(d) Abscheiden einer Schicht aus Siliciumoxid, die an die erste Schicht angrenzt.

**19.** Behälter gemäß Anspruch 18, wobei es sich bei der Oxidationsmittelkomponente um Sauerstoff, Distickstoffoxid, Kohlendioxid, Luft oder eine inerte Verbindung handelt.

**20.** Behälter gemäß Anspruch 19, wobei das Kunststoffsubstrat abgesehen von dem Kontakt mit dem eingeschlossenen Plasma gegenüber der Kammer elektrisch isoliert ist.

**21.** Behälter gemäß Anspruch 1, wobei es sich bei dem Parylenpolymer um Parylen N, Parylen C oder Parylen D handelt.

**22.** Verfahren zur Abscheidung einer nichtidealen Sperrbeschichtungssequenzzusammensetzung auf der äußeren

Oberfläche eines Kunststoffbehälters, umfassend:

(a) Auswählen einer härtbaren Komponente, die i) polyfunktionelle Acrylate oder ii) Gemische von Monoacrylaten und polyfunktionellen Acrylaten umfasst;

(b) Schnellverdampfen der Komponente in die Kammer;

(c) Kondensieren eines Acrylatmaterials aus der verdampften Komponente auf die äußere Oberfläche des Behälters;

(d) Härten des Acrylatmaterials;

(e) Verdampfen einer siliciumorganischen Komponente und Mischen der verflüchtigten siliciumorganischen Komponente mit einer Oxidationsmittelkomponente und gegebenenfalls einer Inertgaskomponente unter Bildung eines Gasstroms außerhalb der Kammer;

(f) Bilden eines Glimmentladungsplasmas in der Kammer aus einer oder mehreren der Gasstromkomponenten;

(g) gesteuertes Strömenlassen des Gasstroms in das Plasma, wobei wenigstens ein Teil des Plasmas darin eingeschlossen wird;

(h) Abscheiden eines Materials aus Siliciumoxid angrenzend an das Acrylatmaterial;

(i) Wiederholen der obigen Schritte (a) bis (d), so dass ein Acrylatmaterial auf dem Siliciumoxidmaterial abgeschieden wird; und

(j) Wiederholen der obigen Schritte (e) bis (h), so dass ein Siliciumoxidmaterial auf dem Acrylatmaterial abgeschieden wird;

(k) Entnehmen des Behälters aus der Kammer und Abscheiden eines organischen Materials, das aus Polyvinylidenchlorid, Vinylidenchlorid-Methylmethacrylat-Methylacrylat-Acrylsäure-Polymer (PVDC), Vinylidenchlorid-Acrylnitril-Methylmethacrylat-Methylacrylat-Acrylsäure-Copolymeren, duroplastischen Epoxymaterialien, Parylenpolymeren oder Polyestern ausgewählt ist, über der nichtidealen Sperrbeschichtungssequenz.

**23.** Verfahren gemäß Anspruch 22, wobei das organische Material von Schritt (k) durch Tauchbeschichtung, Walzenbeschichtung oder Sprühen auf der nichtidealen Sperrbeschichtungssequenz gebildet wird.

**24.** Verfahren gemäß Anspruch 22, wobei Schritt (k) aus dem Auftragen von PVDC durch Tauchbeschichtung auf die nichtideale Sperrbeschichtungszusammensetzungssequenz besteht.

**25.** Verfahren gemäß Anspruch 22, das weiterhin das zwei- bis zwanzigmalige Wiederholen der Schritte (i) bis (j) vor Schritt (k) umfasst.

**26.** Verfahren gemäß Anspruch 22, wobei das Acrylatmaterial mit einem Sauerstoffplasma vorbehandelt wird.

## Revendications

**1.** Récipient en plastique ayant une extrémité ouverte, une extrémité fermée, une surface intérieure et une surface extérieure ; comprenant une séquence de couche barrière non idéale associée sur la surface extérieure de la composition dudit récipient comprenant :

$$\sum_{n} (\text{matériau organique} + \text{matériau inorganique})$$

où n = de 1 à 20,

dans lequel ledit matériau inorganique est déposé sur ledit matériau organique ;

et dans lequel un matériau organique choisi parmi le chlorure de polyvinylidène, le polymère de chlorure de vinylidène-méthacrylate de méthyle-acide acrylique de méthacrylate (PVDC), les copolymères de chlorure de vinylidène-acrylonitrile-méthacrylate de méthyle-méthylacrylate-acide acrylique, des matériaux époxy thermodurcissables, des polymères parylène ou des polyesters est déposé sur la séquence de couche barrière non idéale.

**2.** Récipient selon la revendication 1, dans lequel le taux de perméation de la lamination desdits matériaux organique et inorganique ($\Pi_{oi}$), est inférieur à l'inverse de la somme de l'inverse du taux de perméation à travers ledit matériau inorganique ($\Pi_i$) de ladite séquence et de l'inverse du taux de perméation à travers ledit matériau organique ($\Pi_o$) de ladite séquence.

**3.** Récipient selon la revendication 2, dans lequel ledit taux de perméation de la lamination desdits matériaux organique et inorganique n'est pas un effet additif.

**4.** Récipient selon la revendication 1, dans lequel l'énergie thermique ($\Delta G_T$) de ladite composition de la séquence de couche barrière non idéale est supérieure à l'énergie thermique dudit composé organique ($\Delta G_A$) et dudit composé inorganique ($\Delta G_B$).

**5.** Récipient selon la revendication 1, dans lequel ledit matériau organique est un polymère acrylique ou acrylate fortement réticulé.

**6.** Récipient selon la revendication 1, dans lequel ledit matériau inorganique est un oxyde de métal.

**7.** Récipient selon la revendication 6, dans lequel ledit oxyde de métal est une composition à base d'un oxyde de silicium ou une composition à base d'un oxyde d'aluminium.

**8.** Récipient selon la revendication 7, dans lequel ladite composition à base d'oxyde de silicium est un oxyde de silicium, $SiO_x$, où x est de 1,0 à 2,5.

**9.** Récipient selon la revendication 5, dans lequel l'épaisseur dudit matériau organique est de 0,1 micromètre à 10 micromètres.

**10.** Récipient selon la revendication 9, dans lequel l'épaisseur dudit matériau organique est de 0,5 micromètre à 3 micromètres.

**11.** Récipient selon la revendication 6, dans lequel l'épaisseur dudit oxyde de métal est de 1 µm à 20 µm (de 100 à 2 000 Angstrom).

**12.** Récipient selon la revendication 11, dans lequel l'épaisseur dudit oxyde de métal est de 5 µm à 10 µm (de 500 à 1 000 Angstrom).

**13.** Récipient selon la revendication 1, dans lequel ladite épaisseur dudit chlorure de polyvinylidène est de 2 à 15 micromètres.

**14.** Récipient selon la revendication 13, dans lequel ladite épaisseur dudit chlorure de polyvinylidène est de 3 à 5 micromètres.

**15.** Récipient selon la revendication 1, dans lequel ledit matériau organique est un mélange polymérisé de mono- et di-acrylates.

**16.** Récipient selon la revendication 1, dans lequel ledit matériau organique comprend un acrylate polymérisé et ledit matériau inorganique comprend un oxyde de silicium.

**17.** Récipient selon la revendication 1, dans lequel ledit acrylate dudit matériau organique est déposé sur ladite surface extérieure dudit récipient dans une chambre préalablement mise sous vide comprenant les étapes suivantes :

(a) la mesure d'un composé monomère réticulable dans un système de vaporisation chauffé ;
(b) la vaporisation flash dudit composé dans ladite chambre ;

(c) la condensation, la vaporisation ou l'atomisation d'un film du composé sur la surface extérieure dudit récipient ; et
(d) la réticulation dudit film.

**18.** Récipient selon la revendication 17, dans lequel ledit matériau inorganique est déposé dans ladite chambre préalablement mise sous vide sur ledit matériau organique selon les étapes suivantes :

(a) la vaporisation d'un composé organosilicium et le mélange du composé organosilicium volatilisé avec un composé oxydant et facultativement un gaz inerte pour former un flux de gaz à l'extérieur.de la chambre ;
(b) l'établissement d'un plasma à décharge luminescente dans la chambre à partir d'un ou plusieurs des composés du flux de gaz ;
(c) l'écoulement contrôlé du flux de gaz dans le plasma tout en confinant au moins une portion du plasma en dedans ;
(d) le dépôt d'une couche d'oxyde de silicium adjacente à ladite première couche.

**19.** Récipient selon la revendication 18, dans lequel ledit composé oxydant est un oxyde nitreux d'oxygène, le dioxyde de carbone, l'air ou un composé inerte.

**20.** Récipient selon la revendication 19, dans lequel le substrat en plastique est isolé électriquement de la chambre si ce n'est pour le contact avec le plasma confiné.

**21.** Récipient selon la revendication 1, dans lequel ledit polymère de parylène est le parylène N, le parylène C ou le parylène D.

**22.** Procédé de dépôt d'une composition de séquence de couche barrière non idéale sur la surface extérieure d'un récipient en plastique comprenant :

(a) le choix d'un composé réticulable comprenant : i) des acrylates polyfonctionnels ou ii) des mélanges de monoacrylates et d'acrylates polyfonctionnels ;
(b) la vaporisation flash dudit composé dans ladite chambre ;
(c) la condensation d'un matériau acrylate dudit composé vaporisé sur la surface extérieure dudit récipient ;
(d) la réticulation dudit matériau acrylate ;
(e) la vaporisation d'un composé organosilicium et le mélange d'un composé organosilicium volatilisé avec un composé oxydant et facultativement un composé de gaz inerte pour former un flux de gaz à l'extérieur de la chambre ;
(f) l'établissement d'un plasma à décharge luminescente dans la chambre à partir d'un ou plusieurs des composés du flux de gaz ;
(g) l'écoulement contrôlé du flux de gaz dans le plasma tout en confinant au moins une portion du plasma en dedans ;
(h) le dépôt d'un matériau d'oxyde de silicium adjacent audit matériau acrylate ;
(i) la répétition des étapes de (a) à (d) ci-dessus, pour ainsi déposer un matériau acrylate sur ledit matériau d'oxyde de silicium ; et
(j) la répétition des étapes de (e) à (h) ci-dessus, pour ainsi déposer un matériau d'oxyde de silicium sur ledit matériau acrylate ;
(k) le retrait du récipient de la chambre et le dépôt d'un matériau organique choisi parmi le chlorure de polyvinylidène, le polymère de chlorure de vinylidène-méthacrylate de méthyle-acide acrylique de méthacrylate (PVDC), les copolymères de chlorure de vinylidène-acrylonitrile-méthacrylate de méthyle-méthylacrylate-acide acrylique, des matériaux époxy thermodurcissables, des polymères parylène ou des polyesters sur la séquence de couche barrière non idéale.

**23.** Procédé selon la revendication 22, dans lequel le matériau organique de l'étape (k) est formé sur la séquence de couche barrière non idéale par un revêtement au trempé, un revêtement au rouleau ou par pulvérisation.

**24.** Procédé selon la revendication 22, dans lequel l'étape (k) consiste en un revêtement au trempé de PVDC sur ladite séquence de composition de couche barrière non idéale.

**25.** Procédé selon la revendication 22 comprenant en outre les étapes répétées de (i) à (j) de 2 à 20 fois avant l'étape (k).

**26.** Procédé selon la revendication 22, dans lequel ledit matériau acrylate est prétraité par un plasma d'oxygène.

FIG-1

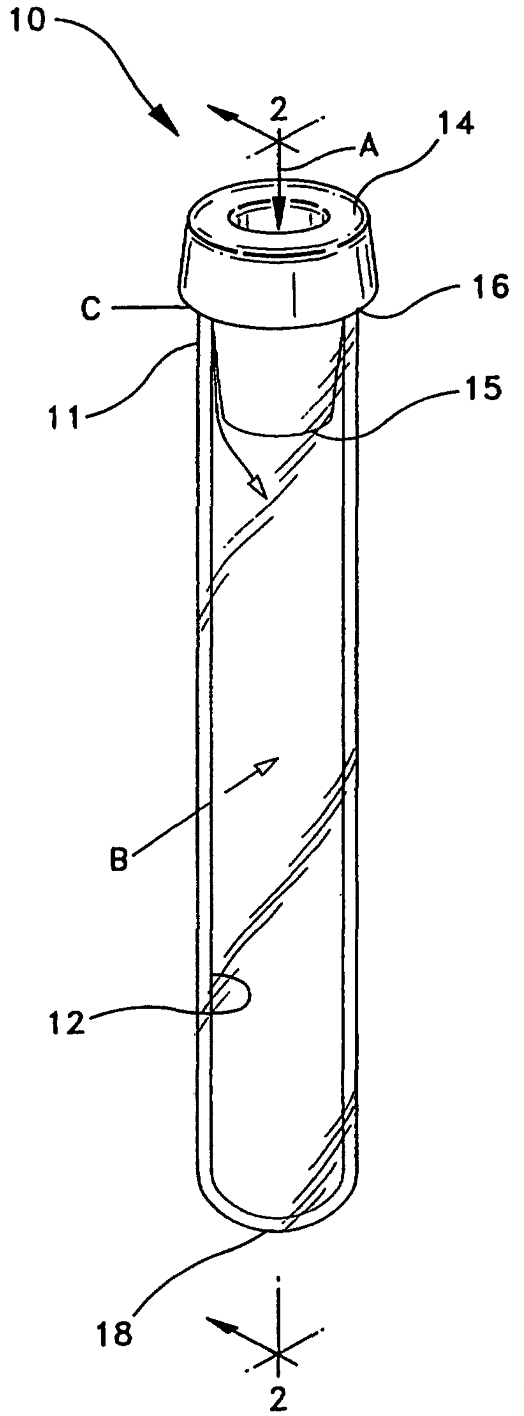


FIG-2

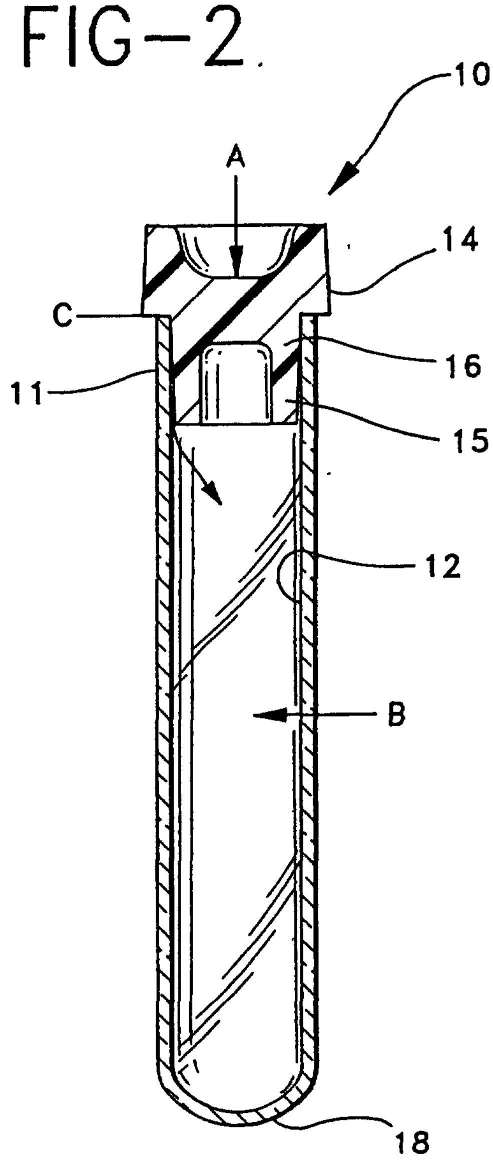

FIG-3

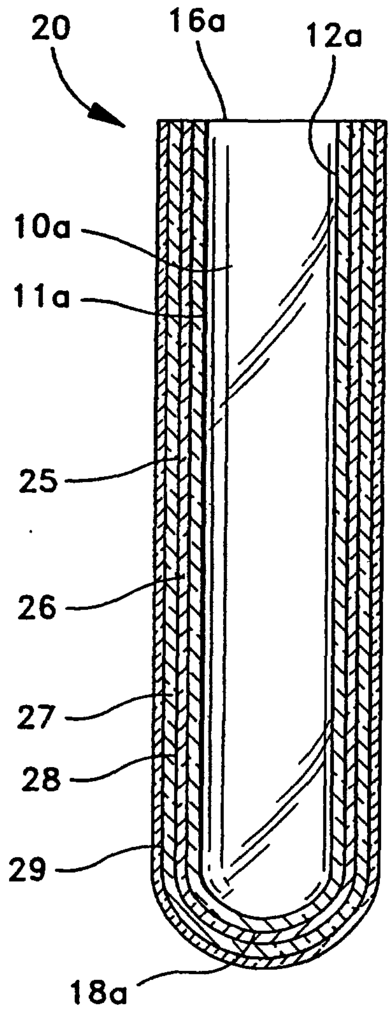
20
16a
12a
10a
11a
25
26
27
28
29
18a

FIG-4

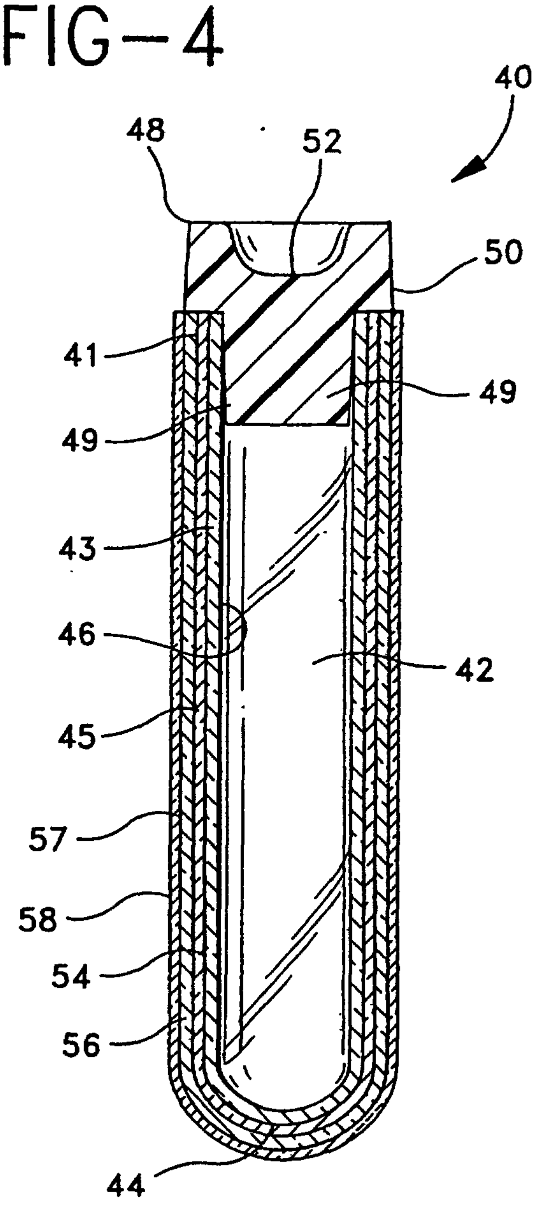
40
48
52
50
41
49
49
43
46
42
45
57
58
54
56
44

FIG-5

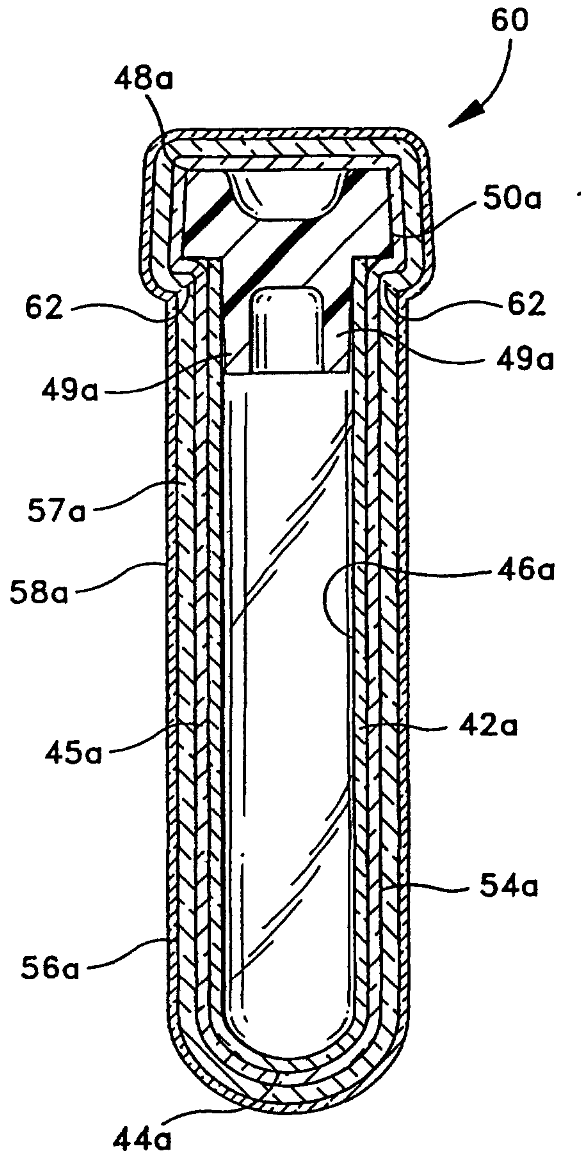

FIG-6

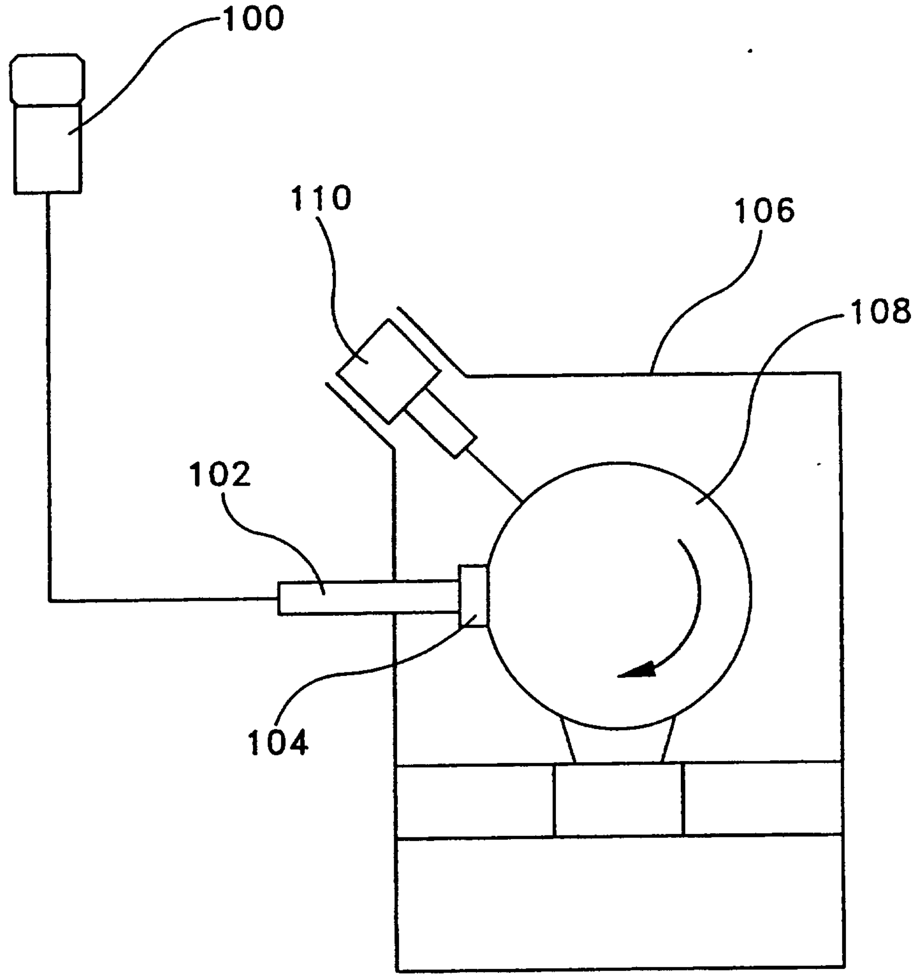

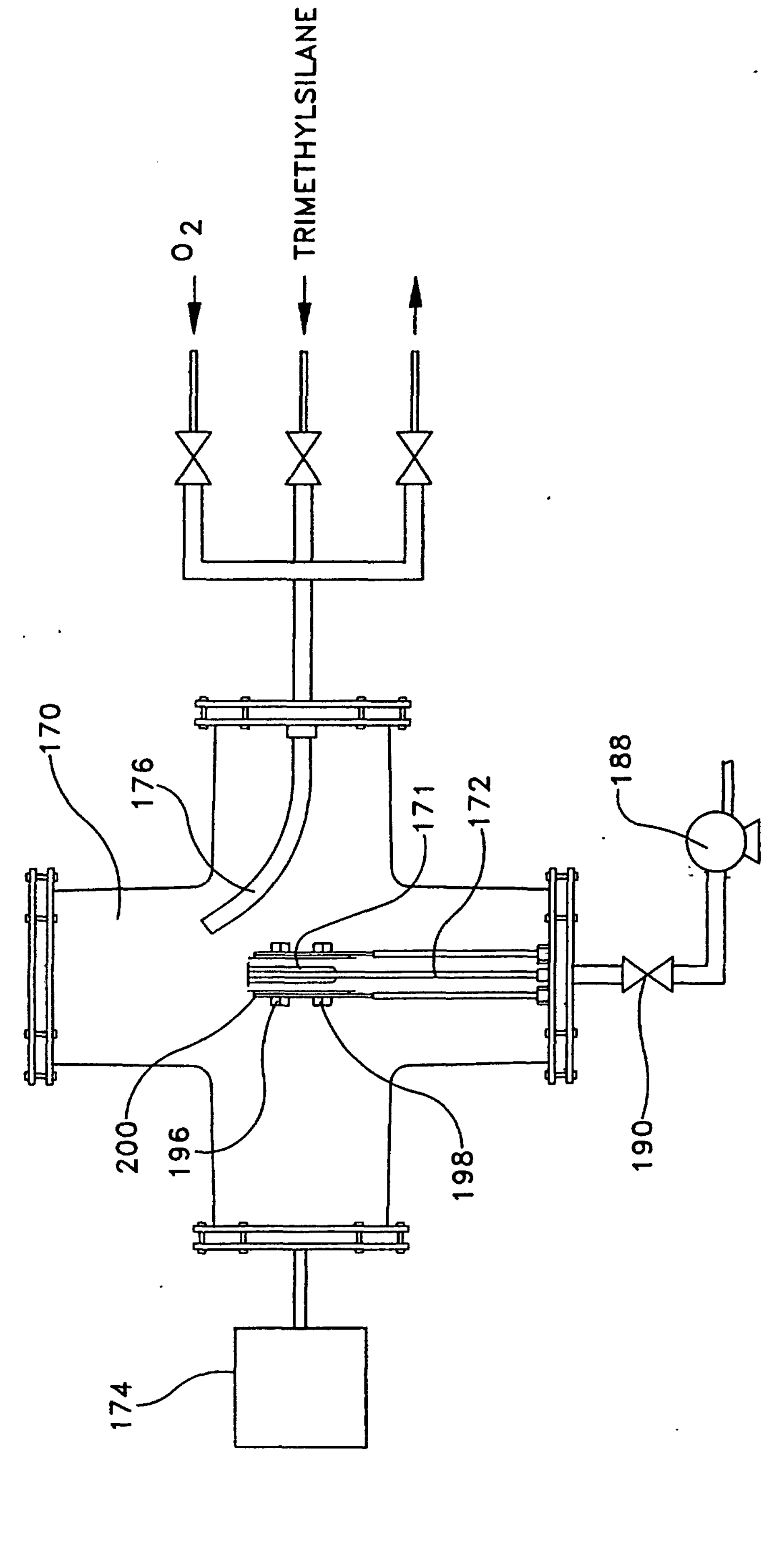


FIG-7

FIG-8

ARRHENIUS PLOT OF OXYGEN TRANSPORT THROUGH PET, PET/SiOx, AND SiOx/PET/SiOx BARRIER FILMS

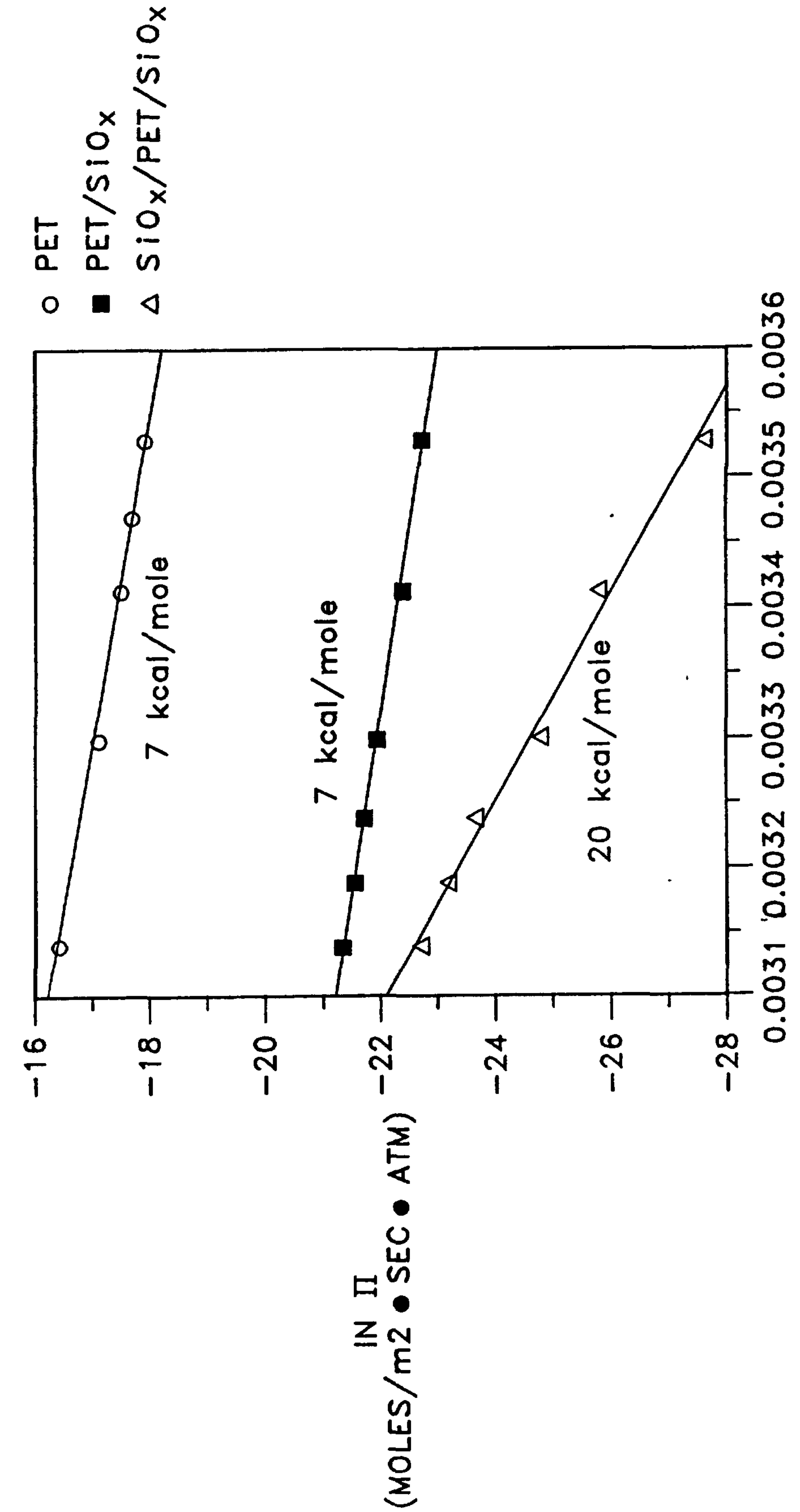

FIG-9 DRAW VOLUME LOSS FOR PET/[Ac/$SiO_x$]$_n$ TUBES AT 40°C, 1 ATM

| n | DRAW LOSS (% PER YEAR) | SHELF LIFE (MONTHS) |
|---|---|---|
| 0 | 41 | 2.9 |
| 1 | 41 | 2.9 |
| 2 | 17 | 7.9 |
| 4 | 12 | 13.7 |